# EUROPEAN PATENT SPECIFICATION

(11) **EP 1 572 715 B1**
(45) Date of publication and mention of the grant of the patent: **22.11.2006**
(21) Application number: 03789347.6
(22) Date of filing: 18.12.2003
(51) Int. Cl.: C07J 7/00, C07J 9/00, A61K 31/57, A61K 31/575, A61P 35/00

(54) **STEROID COMPOUNDS WITH ANTI-TUMOR ACTIVITY**
STEROID-VERBINDUNGEN MIT ANTI-TUMORALER WIRKUNG
COMPOSES STEROIDIENS POSSEDANT UNE ACTIVITE ANTITUMORALE

(30) Priority: 18.12.2002 EP 02447257; 20.12.2002 US 435834 P
(43) Date of publication of application: 14.09.2005
(73) Proprietor: Unibioscreen S.A., 1070 Bruxelles (BE); UNIVERSITE LIBRE DE BRUXELLES, 1050 Bruxelles (BE)
(72) Inventor: BRAEKMAN, Jean-Claude, B-1640 Rhôde-St-Genèse (BE); VAN QUAQUEBEKE, Eric, B-1200 Woluwe St-Lambert (BE); INGRASSIA, Laurent, B-4120 Braine L'Aleud (BE); DEWELLE, Janique, B-6238 Luttre (BE); KISS, Robert, B-1600 Sint-Pieters-Leeuw (BE); DARRO, Francis, B-1180 Uccle (BE)
(74) Representative: Brants, Johan P.E.
(86) International application number: PCT/EP2003/014567
(87) International publication number: WO 2004/055039

(56) References cited:
- JP-A- 6 321 782
- MODI, SANDEEP P. ET AL: "Conjugate addition of Grignard reagents to enones and dienones" JOURNAL OF ORGANIC CHEMISTRY (1989), 54(10), 2317-21, XP002242748
- CIOBANU, L. C. ET AL: "Synthesis and steroid sulfatase inhibitory activity of C19- and C21-steroidal derivatives bearing a benzyl-inhibiting group" EUROPEAN JOURNAL OF MEDICINAL CHEMISTRY (2001), 36(7-8), 659-671, XP004372876
- R. P. BOIVIN ET AL.: "Structure-Activity Relationship of 17alpha-Derivatives of Estradiol as Inhibitors of Steroid Sulfatase" J. MED. CHEM., vol. 43, 2000, pages 4465-4478, XP002232869
- ENDO, YASUYUKI ET AL: "Oxygenated cholesterols as ligands for cytosolic-nuclear tumor promoter binding protein: Yakkasteroids" BIOCHEMICAL AND BIOPHYSICAL RESEARCH COMMUNICATIONS (1993), 194(3), 1529-35, XP002232867
- BERGSTROM, CARL P. ET AL: "Inhibition of cholesterol side-chain cleavage. Part 5. Synthesis of 22-(p-chlorophenyl)cholesterol analogs" DRUG DESIGN AND DELIVERY (1991), 7(4), 259-68, XP001079777

## Description

### Field of the invention

The present invention relates to the medical field. In a first aspect, the present invention relates to novel steroid compounds having a pharmacological activity, in particular an anti-tumor activity. In a second aspect, the present invention relates to a method for preparation of said steroid compounds. The invention further relates in a third aspect to a pharmaceutical composition comprising an effective amount of said steroid compounds. In a fourth aspect, the present invention concerns the use of said steroid compounds as a medicament and the use of said steroid compounds for the preparation of a medicament for the treatment of cancer. In a fifth aspect, the present invention relates to the use of a steroid compound or a pharmaceutical composition comprising said steroid compound according to the invention in the treatment of cancer.

### Background of the invention

Cancer develops in a given tissue when some genomic mutation perturbs cell cycle kinetics by increasing cell proliferation or decreasing cell death, or both. This perturbation leads to unrestrained growth of a genomically transformed cell population. Some cells from this transformed cell population may switch to the angiogenic phenotype, enabling them to recruit endothelial cells from the healthy tissue and leading to the sustained growth of the developing neoplastic tumor tissue. Subsequently, some cells migrate from the neoplastic tumor tissue and colonize new tissues, using blood or lymphatic vessels as major routes of migration. This process is also known as the metastatic process.

In practice, most of the agents used today in hospitals to treat cancer patients are drugs, which more or less directly target the cell kinetics, i.e. cell proliferation, of the cancer to be combated. The working mechanism of such anti-cancer drugs essentially relates to the disruption of the development of malignant cells by acting on cell kinetics. These drugs include alkylating agents, intercalating agents, antimetabolites, etc... most of which target DNA or enzymes regulating the DNA duplication and elongation process. These drugs attack the DNA.

A major drawback of these drugs involves that the drugs do not work in a selective manner, i.e. they do not select between normal and neoplastic cells. They are used in accordance with the fact that the DNA of rapidly proliferating cells, i.e. cancer cells, is more sensitive to this type of agents than the DNA of less rapidly proliferating cells, i.e. normal cells. However, rapidly growing tumors are not always tumors exhibiting high levels of cell proliferation. Rapidly growing tumors may also include tumors which exhibit low levels of cell death compared to the normal cell population from which these tumor cells issue. For these types of rapidly growing tumors, the above-described, non-selective anti-cancer drugs are not effective.

In addition, the great majority of the drugs used in the standard treatment of cancer using the cell kinetics approach have the drawback of being toxic or even highly toxic, i.e. involving many detrimental side-effects on healthy cells, tissues and organs, and this limits their clinical use to a relatively low number of administrations per patient. In addition, several of these compounds must be combined into a poly-chemotherapeutic regimen in order to have any observable effect against cancer. By way of evidence such anti-cancer drug combinations increase detrimentally the toxicity of the treatment and also limit the number of administrations that can be applied.

Some anti-cancer drugs from natural origins, such as e.g. anti-tubulin compounds, using a therapeutic approach different from the cell kinetics approach, have been proposed. Said drugs aim to prevent the migration of cancer cells which escape from the primary tumor tissue and first invade neighbouring tissue therefore establishing metastases. However, the compounds of this type known so far also show major toxic side effects, which limits their use over long periods of treatment.

Therefore, there remains an urgent need in the art for finding improved anti-cancer drugs, which overcome at least some of the above-mentioned drawbacks. Consequently, it is a general object of the invention to provide im proved anti-cancer drugs. More in particular, it is an object of the present invention to provide novel anti-cancer drugs and methods for synthesizing these. It is still another object of the invention to provide intermediate compounds as a result of the aforementioned synthesis methods, which have a pharmaceutical utility, e.g. in the treatment of cancer.

### Summary of the invention

In a first aspect the present invention relates to steroid compounds having the below given basic structure and being substituted in position B.

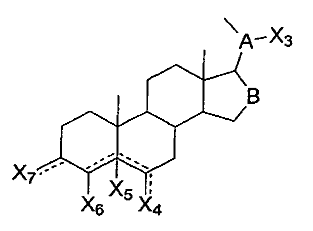

In particular, the present invention relates to steroid compounds of formula IB or pharmaceutically acceptable salts, stereo isomeric forms or racemic mixtures thereof. 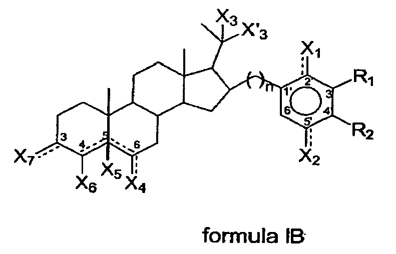 wherein n, X₁, X₂, X₃, X₃', X₄, X₅, X₆, X₇, R₁, R₂ are as defined in claim 1. The present invention provides novel steroid compounds that have anti-tumor activity and that are consequently very suitable for use in all kind of therapeutic applications as described below.

In a second aspect, the present invention relates to a method for synthesizing said steroid compounds.

In addition, the present invention further relates to pharmaceutical compositions comprising the above-described compounds. Furthermore, the present invention relates to steroid compounds for use as a medicament and for use in the preparation of a medicament for the treatment of diseases associated with cell proliferation, in particular for treatment of cancer. The present invention further relates to the use of the above-described compounds or a pharmaceutical composition comprising said compounds in the treatment of cancer.

### Detailed description of the figures

Figure 1 represents an example of a reaction scheme for preparing a steroid compound according to the invention.

Figures 2 and 3 represent the anti-tumor activity of different steroid compounds according to the invention on six human cancer cell lines. Figure 2 and 3 represent the anti-tumor activity of compounds UBS881 and UBS1664, respectively.

Figure 4 compares the cytotoxic and anti-tumor activity of different compounds according to the invention, in particular compounds UBS881, UBS1664, on six human cancer cell lines.

### Detailed description of the invention

### Steroid compounds according to the invention

A tot of steroids compounds are described in the literature. These compounds have various biological activities. For example, WO 96/10031 and WO 98/14194 describe steroid derivatives as neurochemical stimulators of a specific neuroepithelial receptor to alleviate symptoms of anxiety.

The present invention now relates to novel steroid compounds showing anti-tumor activity. According to the present invention the term "anti-tumor activity", refers to the *in vitro* as well as *in vivo* anti-tumor effects exerted by the steroid compounds according to the invention. The anti-tumor effects essentially include but are not limited to a dramatic decrease of cell growth and a pro-apoptotic effect. Importantly, the steroid compounds according to the invention exhibits anti-tumor activity on a large number of cancer types, such as but not limited to glioma cancer, colon cancer, lung cancer and bladder cancer amongst others.

Importantly, the steroid compounds according to the invention also have an anti-migratory effect. Migration refers to the process whereby cells migrate from the neoplastic tumor tissue and colonize new tissues, using blood or lymphatic vessels as major routes of migration. This process is also known as the metastatic process. According to the present invention the term "anti-migratory", refers to the ability of compounds according to the invention to stop the migration of cells away from the neoplastic tumor tissue and thus reduces the colonization of newtissues by these cells.

The term "steroid" as used herein is intended to mean compounds having a perhydrogenated cyclopentanophenanthrene nucleus. The compounds according to the invention, represented by the general formula given below, have four rings, represented by the letters A to D.

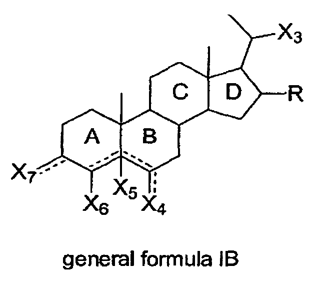

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent

As used herein the term "glycosylated" or "glycosyl" refers a saccharyl moiety such as a mono-, di-, oligo- or an poly-saccharide moiety, a hydroxy-substituted cyclohexyl moiety, the amino derivatives thereof, the thio derivatives thereof or the hydroxyl-protected derivatives thereof such as acetate derivatives thereof. The term "saccharyl" as used herein refers to a saccharide moiety which comprises monosaccharides, di-, tri-, oligo- and polysaccharides. Exemplary monosaccharide moiety includes but is not limited to a pentosyl, a hexosyl, or a heptosyl moiety. The glycosyl moiety may also be substituted with various groups. Such substitutions may include lower alkyl, lower alkoxy, acyl, carboxy, carboxyamino, amino, acetamido, halo, thio, nitro, keto, and phosphatyl groups, wherein the substitution may be at one or more positions on the saccharide. Moreover, the glycosyl may also be present as a deoxy glycosyl. The hydroxy-substituted cyclohexyl moiety includes but is not limited to mono-hydroxycyclohexyl group such as 2-, 3- or 4-hydroxycyclohexyl group, a di-hydroxycyclohexyl group such as 2,3-, 2,4-, 2,5-, 2,6-, 3,4-, or 3,5 -dihydroxycyclohexyl) group, a tri-hydroxycyclohexyt group such as 2,3,4-, 2,3,5-, 2,3,6-, 3,4,5-, or 3,4,6-trihydroxycyclohexyl group or a tetra-hydroxycyclohexyl group such as 2,3,4,5-, 2,3,4,6-, or 2,3,5,6-tetrahydroxycyclohexyl group, hydroxyl-protected derivatives thereof, thio derivatives thereof or amino derivatives thereof.

In an embodiment, said glycosyl is a saccharyl moiety, a hydroxy-substituted cyclohexyl moiety, including monosaccharide, L or D isomers thereof, α or β form thereof, pyranose or furanose form thereof, combination thereof, deoxy derivatives thereof, hydroxyl-protected acetate derivatives thereof, amino derivatives thereof optionally substituted, thio derivatives thereof, di-, tri-, oligo- and polysaccharide thereof.

As used herein, the term "oxo" or "=O" forms a carbonyl moiety with the carbon atom to which it is attached. As used herein, the term "carboxyl" or "-COOH" is an acid moiety whereby the carbon atom binds to the carbon atom to which it is attached.

Whenever used in the present invention the term "compounds of the invention" or "steroid compounds" or a similar term is meant to include the compounds of formula IB and any subgroup thereof. This term also refers to the compounds as depicted in Table A and their *N*-oxides, salts, stereoisomeric forms, racemic mixtures, pro-drugs, esters and metabolites, as well as their quatemized nitrogen analogues. The *N-*oxide forms of said compounds are meant to comprise compounds wherein one or several nitrogen atoms are oxidized to the so-called *N*-oxide.

The term "pro-drug" as used herein means the pharmacologically acceptable derivatives such as esters, amides and phosphates, such that the resulting *in vivo* biotransformation product of the derivative is the active drug. The reference by Goodman and Gilman (The Pharmacological Basis of Therapeutics, 8th Ed, McGraw-Hill, Int. Ed. 1992, "Biotransformation of Drugs", p 13-15) describing pro-drugs generally is hereby incorporated. Pro-drugs of the compounds of the invention can be prepared by modifying functional groups present in said component in such a way that the modifications are cleaved, either in routine manipulation or *in vivo,* to the parent component. Typical examples of pro-drugs are described for instance in WO 99/33795, WO 99/33815, WO 99/33793 and WO 99/33792 all incorporated herein by reference. Pro-drugs are characterized by increased bio-availability and are readily metabolized into the active inhibitors *in vivo.*

The compounds according to the invention may also exist in their tautomeric forms. Such forms, although not explicitly indicated in the compounds described herein, are intended to be included within the scope of the present invention.

The term "stereochemically isomeric forms of the analogues according to the invention", as used herein, defines all possible compounds made up of the same atoms bonded by the same sequence of bonds but having different three-dimensional structures which are not interchangeable, which the compounds of the present invention may possess. Unless otherwise mentioned or indicated, the chemical designation of a compound herein encompasses the mixture of all possible stereochemically isomeric forms, which said compound may possess. Said mixture may contain all diastereomers and/or enantiomers of the basic molecular structure of said compound. All stereochemically isomeric forms of the compounds of the invention either in pure form or in admixture with each other are intended to fall within the scope of the present invention.

For therapeutic use, the salts of the compounds according to the invention are those wherein the counter-ion is pharmaceutically or physiologically acceptable.

The pharmaceutically acceptable salts of the compounds according to the invention, i.e. in the form of water-, oil-soluble, or dispersible products, include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such a sarginine, lysine, and so forth. Also, the basic nitrogen-containing groups may be quatemized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

In an embodiment the present invention relates to a steroid compound of the formula IB as indicated above, or a pharmaceutically acceptable salt thereof, wherein X₁, X₂, X₃, X₃', X₄, X₅, X₆, X₇, R₁, R₂ and n are as defined in claim 2.

In another preferred embodiment, the compound according to the invention is a compound having the formula IB as indicated above, or a pharmaceutically acceptable salt thereof, wherein X₁ and X₂ are -OMe, wherein R₁ and R₂ are -H, wherein X₄ and X₇ are oxo, wherein X₃ participates together with X₃' to an oxo functional group, wherein X₅ participates to a double bond between the carbon atoms in position 4 and 5, wherein X₆ is hydrogen, and wherein n is 0.

In another embodiment, the invention relates to a compound having formula IB, wherein X₁, X₂, X₃, X₃', X₄, X₅, X₆, X₇, R₁, R₂ and n are as defined as claim 11.

The compounds according to the invention show cytotoxic activities, which implies that they may be used in various medical applications. As is demonstrated in the examples provided below, the compounds according to the invention have *in vitro* anti-tumor activity.

Furthermore, the compounds according to the invention exhibit a low toxicity level. The terms "toxicity" or "toxic effects" as used herein refer to the detrimental effect(s) a compound may have on healthy cells, tissues or organs. The toxicity level of the compounds according to the invention is surprisingly low. The compounds according to the invention combine the essential features of a good anti-tumor activity and a low level of toxicity. Consequently the compounds according to the invention may be used in pharmaceutical compositions for the treatment of various diseases. In addition, because they have a low level of toxicity the compounds according to the invention may be used during longer periods of treatments.

In addition, the compounds according to the invention also have a show an anti-migratory effect. The compounds according to the invention have the ability to stop the migration of cells away from the neoplastic tumor tissue and thus enable to reduce the colonization of new tissues by these cells.

### Method of preparation

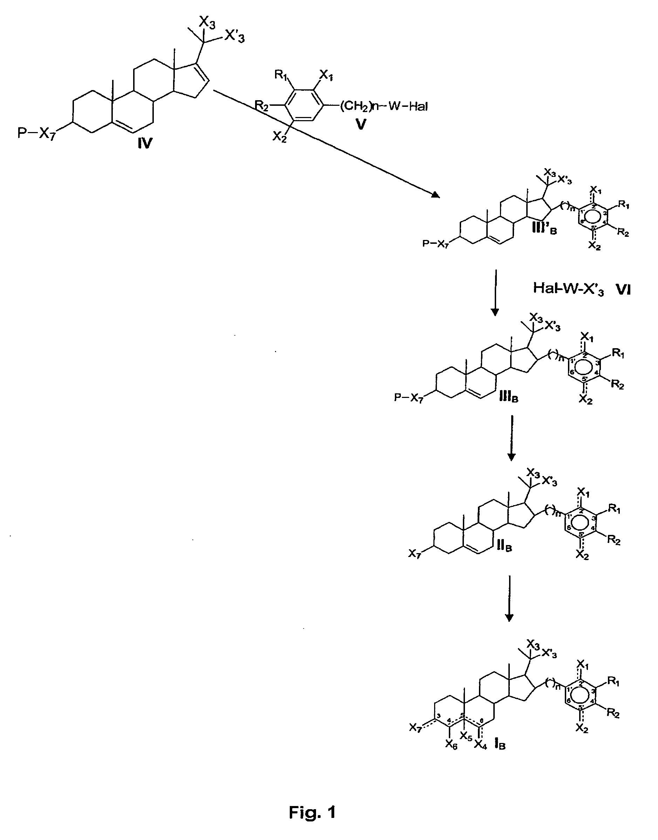

In another embodiment, the present invention relates to methods for preparing the compounds according to the invention. Figure 1 represents a scheme of the methods of preparation according to the invention.

### Formula IB

In another embodiment, the invention relates to a method for synthesizing a compound having the structural formula IB 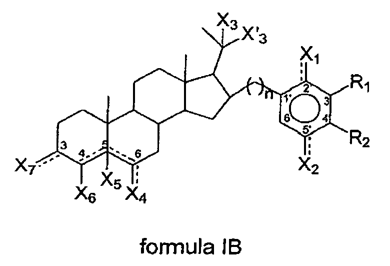 wherein X₁, X₂, X₃, X₃', X₄, X₅, X₆, X₇, R₁, R₂ and n are selected from the group as indicated above, said method comprising the steps of
a) providing a starting material having the structural formula IV, 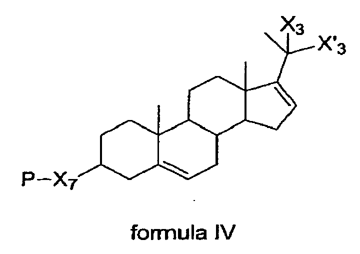 wherein X₃, X₃' and X₇ are selected from the group as indicated above,
   wherein P is a protecting group selected from the group comprising alkyl aryl silane, alkyl silane and carbonylalkylaryl, and wherein P preferably is t-butyl diphenyl silane,
b) effecting reaction between the compound of step a) with an organometallic compound having the structural formula V 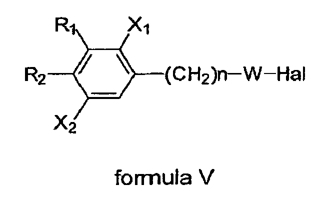 wherein X₁, X₂, R₁, R₂ and n are selected from the group as indicated above, wherein W is a metal or a combination of metals selected from the group comprising magnesium and copper and wherein Hal is a halogen atom, and preferably selected from the group comprising bromine, chlorine and iodine,
   to result in an intermediate having the structural formula III'B 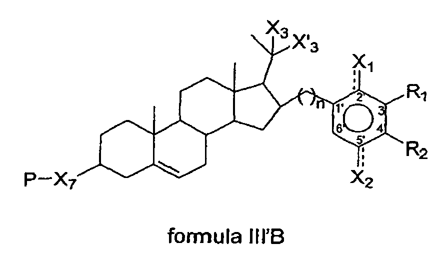 wherein X₁, X₂, X₃, X₃', X₇ R₁, R₂ and n are selected from the group as indicated above, and wherein P is a protecting group as indicated above,
c) effecting reaction between the compound of step b) with an organometallic compound having the structural formula VI

   Hal-W-X'₃ formula VI

   wherein X'₃ is selected from the group as indicated above, wherein W is a metal or a combination of metals selected from the group comprising magnesium and copper, and wherein Hal is a halogen atom, preferably selected from the group comprising bromine, chlorine and iodine,
   to result in an intermediate having the structural formula IIIB 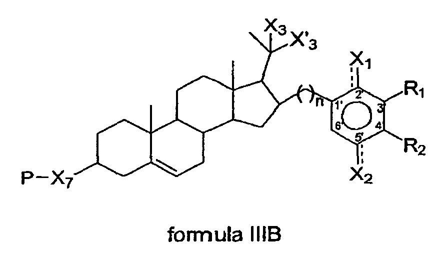 wherein X₁, X₂, X₃, X'₃ X₇, R₁, R₂ and n are selected from the group as indicated above, wherein p is a protecting group,
d) deprotecting the X₇ group of the compound obtained in step c) to form an compound having the structural formula liB 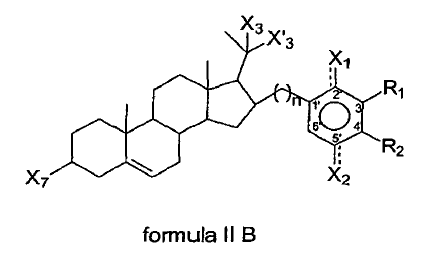 wherein X₁, X₂, X₃, X'₃, X₇, R₁, R₂ and n are selected from the group as indicated above, and
e) oxidizing by reaction with a suitable oxidizing agent or agents to form a compound of formula IB
   or
e) coupling an O-protected glycosyl or non-protected glycosyl to form a compound of formula IIB wherein X₁, X₂, X₃, X'₃, X₇, R₁, R₂ and n are selected from the group as indicated above and X₇ is an O-protected glycosyl or a non-protected glycosyl, and
f) deprotecting the O-protected groups of glycosyl to form the compound having the formula IB wherein X₁, X₂, X₃, X'₃, X₄, X₅, X₆, X₇, R₁, R₂ and n are selected from the group as indicated above.

In another embodiment of the present invention, step (c) consists of reacting the compound of step b) with an O-protected glycosyl or non-protected glycosyl to result in an intermediate having the structural formula IIIB wherein X₁, X₂, X₃, X₇, R₁, R₂ and n are selected from the group as indicated above, wherein P is a protecting group, and wherein X'₃ is an O-protected glycosyl or a non protected glycosyl. The reaction may then proceed with steps (d) and eventually (e) as described above.

Protected forms of the inventive compounds are included within the scope of the present invention. A variety of protecting groups are disclosed, for example, in T. H. Greene and P. G. M. Wuts, Protective Groups in Organic Synthesis, Third Edition, John Wiley & Sons, New York (1999), which is incorporated herein by reference in its entirety. For example, hydroxy protected forms of the inventive compounds are those where at least one of the hydroxyl groups is protected by a hydroxy protecting group. Illustrative hydroxyl protecting groups include but not limited to tetrahydropyranyl; benzyl; methylthiomethyl; ethylthiomethyl; pivaloyl; phenylsulfonyl; triphenylmethyl; trisubstituted silyl such as trimethyl silyl, triethylsilyl, tributylsilyl, tri-isopropylsilyl, t-butyldimethylsilyl, tri-t-butylsilyl, methyldiphenylsilyl, ethyldiphenylsilyl, t-butyldiphenylsilyl and the like; acyl and aroyl such as acetyl, pivaloylbenzoyl, 4-methoxybenzoyl, 4-nitrobenzoyl and aliphatic acylaryl and the like. Keto groups in the inventive compounds may similarly be protected.

The steroid compounds according to the present invention are prepared using an enone as the starting compound. These enones, having general formula IV, can be synthesised according to the procedure described in Tetrahedron, 1993, 49(23), 5079-5090. The derivatives represented by formula V or formula VI are prepared either from corresponding commercially available halides or by known methods as described for instance in Tetrahedron, 1982, 3555-3561. Example 2 provided below illustrates the preparation of several different steroid compounds according to the invention

In another embodiment, the present invention also relates to a compound, which is obtained by any of the steps according to the above-described methods for synthesis of a compound of formula IB. A number of these compounds identified herein as intermediates also find utility as pharmaceutical agents. Certain intermediate compounds obtained in any of the above-described steps of the synthesis methods may be useful in the treatment disorders, in particular cancers.

### Uses of the compounds according to the invention

An important feature attributed to the compounds according to the invention is their broad application possibility. The compounds according to the invention exhibit anti-tumor activity on a broad panel of histological tumor types. As will be shown in the examples described below, the compounds according to the invention exert significant anti-tumor effects on several tumor models tested, including glioma, colon, lung and bladder cancer (see e.g. example 3).

In addition, the compounds according to the invention also exhibit anti-migratory effect on cancer cells, as illustrated in example 4 provided below.

When a malignant tumour has reached a certain size, tumour cells move away from the initial tumour site and start to migrate. The actin cytoskeleton, tubulin and adhesion molecules linking the constituents of extracellular matrix to intracellular actin cytoskeleton are central to locomotion. The extracellular matrix proteins such as fibronectin, laminin and collagen are recognized by endogenous lectins which specifically bind to various sugar moieties (β-galactoside, fucose, manose, etc) present in said proteins. For example, the selectins and their ligands (fucose-related Lewis antigens) play critical roles in the invasion processes of various types of cancers (including those of the stomach, lung and melanomas) towards the liver. Various Lewis antigen types also exert significant roles in neoangiogenesis processes. This selectin/Lewis antigen system therefore represents new potential therapeutic targets in cancer field. For example, an increased expression of sialyl Lewis antigen correlates with poor survival in patients with colorectal carcinoma (Nakamori *et al,* 1993), an increased expression of Lewis^{x} antigen correlates with metastatic potential and poor prognostic in patients with gastric carcinoma (Mayer *et al,* 1996). Some of the compounds of the invention are believed to bind to the selectin of tumour cells thereby preventing said cells to migrate to site comprising the Lewis antigen. Other compounds of the invention are believed to bind to other lectins, including for example galectins or manose binding proteins.

Due to these interesting properties; in particular the anti-tumor activity, the anti-migratory effect and the low level of toxicity, the steroid compounds according to the invention are particularly suitable for use as a medicament in the treatment of diseases associated with cell proliferation and cell migration, and even in particular in the treatment of cancer. Therefore, in another embodiment, the invention relates to compounds according to the invention for use as a medicament. In yet another embodiment, the invention provides compounds for use in the preparation of a medicament for treating cancer.

The term "diseases associated with cell proliferation and cell migration" as used herein refers to, but is not limited to, any type of cancer or condition involving cell proliferation and cell migration, including for example chronic inflammation and restenosis in cardiovascular disease. The compounds of the invention may be especially used in the treatment of cancers such as but not limited to leukemia, non-small cell lung cancer, small cell lung cancer, CNS cancer, melanoma, ovarian cancer, renal cancer, prostate cancer, breast cancer, glioma, colon cancer, bladder cancer, sarcoma, pancreatic cancer, colorectal cancer, head and neck cancer, liver cancer and hematological cancer and lymphoma.

In addition, the compounds according to the invention may also be very suitable in the treatment of scar tissue and wounds. It is believed that most, if not all, of the compounds of the present invention can act as active ingredients in treating scar tissue and in promoting wound healing and tissue regeneration.

### Pharmaceutical compositions comprising the steroid compounds

In another embodiment, the present invention relates to a pharmaceutical composition comprising a pharmaceutically acceptable excipient and a therapeutic amount of a compound according to the invention.

The term "therapeutically effective amount" as used herein means that amount of active compound or component or pharmaceutical agent that elicits the biological or medicinal response in a tissue, system, animal or human that is being sought by a researcher, veterinarian, medical doctor or other clinician, which includes alleviation of the symptoms of the disease being treated.

The pharmaceutical composition can be prepared in a manner known per se to one of skill in the art. For this purpose, at least one compound having formula IB, one or more solid or liquid pharmaceutical excipients and, if desired, in combination with other pharmaceutical active compounds, are brought into a suitable administration form or dosage form which can then be used as a pharmaceutical in human medicine or veterinary medicine.

Particular forms of the pharmaceutical composition may be, for example, solutions, suspensions, emulsions, creams, tablets, capsules, nasal sprays, liposomes or micro-reservoirs, especially compositions in orally ingestible or sterile injectable form, for example, as sterile injectable aqueous or oleaginous suspensions or suppositories. The preferred form of composition contemplated is the dry solid form, which includes capsules, granules, tablets, pills, boluses and powders. The solid carrier may comprise one or more excipients, e.g. lactose, fillers, disintegrating agents, binders, e.g. cellulose, carboxymethylcellulose or starch or anti-stick agents, e.g. magnesium stearate, to prevent tablets from adhering to tabletting equipment Tablets, pills and boluses may be formed so as to disintegrate rapidly or to provide slow release of the active ingredient

In order to enhance the solubility and/or the stability of the compounds of a pharmaceutical composition according to the invention, it can be advantageous to employ α-, β- or γ-cyclodextrins or their derivatives. In addition, co-solvents such as alcohols may improve the solubility and/or the stability of the compounds. In the preparation of aqueous compositions, addition of salts of the compounds of the invention are obviously more suitable due to their increased water solubility.

Appropriate cyclodextrins are α-, β- or γ-cyclodextrins (CDs) or ethers and mixed ethers thereof wherein one or more of the hydroxy groups of the anhydroglucose units of the cyclodextrin are substituted with alkyl, particularly methyl, ethyl or isopropyl, e.g. randomly methylated β-CD; hydroxyalkyl, particularly hydroxyethyl, hydroxypropyl or hydroxybutyl; carboxyalkyl, particularly carboxymethyl or carboxyethyl; alkylcarbonyl, particularly acetyl; alkyloxycarbonylalkyl or carboxyalkyloxyalkyl, particularly carboxymethoxypropyl or carboxyethoxypropyl; alkylcarbonyloxyalkyl, particularly 2-acetyloxypropyl. Especially noteworthy as complexants and/or solubilizers are β-CD, randomly methylated β-CD, 2,6-dimethyl- β-CD, 2-hydroxyethyl-β-CD, 2-hydroxyethyl-γ-CD, 2-hydroxypropyl-γ-CD and (2-carboxymethoxy)propyl- β-CD, and in particular 2-hydroxypropyl- β-CD (2-HP- β-CD). The term mixed ether denotes cyclodextrin derivatives wherein at least two cyclodextrin hydroxy groups are etherified with different groups such as, for example, hydroxypropyl and hydroxyethyl. An interesting way of formulating the analogues in combination with a cyclodextrin or a derivative thereof has been described in EP-A-721,331. Although the formulations described therein are with antifungal active ingredients, they are equally interesting for formulating the analogues. Said formulations may also be rendered more palatable by adding pharmaceutically acceptable sweeteners and/or flavors.

More in particular, the compositions may be formulated in a pharmaceutical formulation comprising a therapeutically effective amount of particles consisting of a solid dispersion of the compounds of the invention and one or more pharmaceutically acceptable water-soluble polymers.

The term "a solid dispersion" defines a system in a solid state (as opposed to a liquid or gaseous state) comprising at least two components, wherein one component is dispersed more or less evenly throughout the other component or components. When said dispersion of the components is such that the system is chemically and physically uniform or homogenous throughout or consists of one phase as defined in thermodynamics, such a solid dispersion is referred to as "a solid solution". Solid solutions are preferred physical systems because the components therein are usually readily bioavailable to the organisms to which they are administered. The term "a solid dispersion" also comprises dispersions that are less homogenous throughout than solid solutions. Such dispersions are not chemically and physically uniform throughout or comprise more than one phase.

The water-soluble polymer is conveniently a polymer that has an apparent viscosity of 1 to 100 mPa.s when dissolved in a 2 % aqueous solution at 20°C solution. Preferred water-soluble polymers are hydroxypropyl methylcelluloses or HPMC. HPMC having a methoxy degree of substitution from about 0.8 to about 2.5 and a hydroxypropyl molar substitution from about 0.05 to about 3.0 are generally water soluble. Methoxy degree of substitution refers to the average number of methyl ether groups present per anhydroglucose unit of the cellulose molecule. Hydroxy-propyl molar substitution refers to the average number of moles of propylene oxide which have reacted with each anhydroglucose unit of the cellulose molecule. Various techniques exist for preparing solid dispersions including melt-extrusion, spray-drying and solution-evaporation, melt-extrusion being preferred.

It may further be convenient to formulate the analogues in the form of nanoparticles which have a surface modifier adsorbed on the surface thereof in an amount sufficient to maintain an effective average particle size of less than 1000 nm. Suitable surface modifiers can preferably be selected from known organic and inorganic pharmaceutical excipients. Such excipients include various polymers, low molecular weight oligomers, natural products and surfactants. Preferred surface modifiers include nonionic and anionic surfactants.

Yet another interesting way of formulating the compounds according to the invention involves a pharmaceutical composition whereby the compounds are incorporated in hydrophilic polymers and applying this mixture as a coat film over many small beads, thus yielding a composition with good bio-availability which can conveniently be manufactured and which is suitable for preparing pharmaceutical dosage forms for oral administration. Said beads comprise (a) a central, rounded or spherical core, (b) a coating film of a hydrophilic polymer and an antiretroviral agent and (c) a seal-coating polymer layer. Materials suitable for use as cores in the beads are manifold, provided that said materials are pharmaceutically acceptable and have appropriate dimensions and firmness. Examples of such materials are polymers, inorganic substances, organic substances, and saccharides and derivatives thereof.

### Methods of treatment

As indicated above, due to their favourable anti-tumor properties of the compounds according to the present invention are particularly useful in the treatment of individuals suffering from cancer. Therefore, in another embodiment, the present invention also relates to the use of the steroid compounds according to the invention or to a pharmaceutical composition comprising said steroid compounds in the treatment of cancer. A method of treating cancer comprises administering to an individual in need of such treatment a pharmaceutical composition comprising the steroid compounds according to the invention.

For these purposes, the pharmaceutical composition of the present invention may be administered orally, parenterally, i.e. including subcutaneous injections, intravenous, intramuscular, intrasternal injection or infusion techniques, by inhalation spray, or rectally, in dosage unit formulations containing conventional non-toxic pharmaceutically acceptable carriers, adjuvants and vehicles.

In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

Essentially, the primary modes of treatment of solid tumor cancers comprise surgery, radiation therapy and chemotherapy, separately and in combination. The compounds according to the invention are suitable for use in combination with these medicinal techniques. The compounds of the invention may be useful in increasing the sensitivity of tumor cells to radiation in radiotherapy and also in potentiating or enhancing damage to tumors by chemotherapeutic agents. The compounds and their pharmaceutically acceptable salts may also be useful for sensitising multidrug-resistant tumor cells. The compounds according to the invention are useful therapeutic compounds for administration in conjunction with other DNA-damaging cytotoxic drugs or radiation used in radiotherapy to potentiate their effect.

In another embodiment of the method of the invention, the administration may be performed with food, e.g., a high-fat meal. The term 'with food' means the consumption of a meal either during or no more than about one hour before or after administration of a pharmaceutical composition according to the invention.

For an oral administration form, the compositions of the present invention can be mixed with suitable additives, such as excipients, stabilizers or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose, or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, ethanol, sugar solutions, or mixtures thereof. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

The oral administration of a pharmaceutical composition comprising a steroid compound according to the invention, or a pharmaceutically acceptable salt or ester thereof, is suitably accomplished by uniformly and intimately blending together a suitable amount of the steroid compound in the form of a powder, optionally also including a finely divided solid carrier, and encapsulating the blend in, for example, a hard gelatin capsule. The solid carrier can include one or more substances, which act as binders, lubricants, disintegrating agents, coloring agents, and the like. Suitable solid carriers include, for example, calcium phosphate, magnesium stearate, talc, sugars, lactose, dextrin, starch, gelatin, cellulose, polyvinylpyrrolidine, low melting waxes and ion exchange resins.

Oral administration of a pharmaceutical composition comprising an steroid compound according to the invention, or a pharmaceutically acceptable salt or ester thereof can also be accomplished by preparing capsules or tablets containing the desired amount of the steroid compound, optionally blended with a solid carrier as described above. Compressed tablets containing the pharmaceutical composition of the invention can be prepared by uniformly and intimately mixing the active ingredient with a solid carrier such as described above to provide a mixture having the necessary compression properties, and then compacting the mixture in a suitable machine to the shape and size desired. Molded tablets maybe made by molding in a suitable machine, a mixture of powdered steroid compound moistened with an inert liquid diluent.

When administered by nasal aerosol or inhalation, these compositions may be prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. Suitable pharmaceutical formulations for administration in the form of aerosols or sprays are, for example, solutions, suspensions or emulsions of the compounds of the invention or their physiologically tolerable salts in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant.

For subcutaneous or intravenous administration, the active analogue, if desired with the substances customary therefor such as solubilizers, emulsifiers or further auxiliaries, are brought into solution, suspension, or emulsion. The compounds of the invention can also be lyophilized and the lyophilizates obtained used, for example, for the production of injection or infusion preparations. Suitable solvents are, for example, water, physiological saline solution or alcohols, e.g. ethanol, propanol, glycerol, in addition also sugar solutions such as glucose or mannitol solutions, or alternatively mixtures of the various solvents mentioned. The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these formulations may be prepared by mixing the compounds according to the invention with a suitable non-irritating excipient, such as cocoa butter, synthetic glyceride esters or polyethylene glycols, which are solid at ordinary temperatures, but liquidity and/or dissolve in the rectal cavity to release the drug.

The pharmaceutical compositions of this invention can be administered to humans in dosage ranges specific for each analogue comprised in said compositions. The compounds comprised in said composition can be administered together or separately.

It will be understood, however, that specific dose level and frequency of dosage for any particular patient may be varied and will depend upon a variety of factors including the activity of the specific analogue employed, the metabolic stability and length of action of that compound, the age, body weight, general health, sex, diet, mode and time of administration, rate of excretion, drug combination, the severity of the particular condition, and the host undergoing therapy.

The following examples are meant to illustrate the present invention. These examples are presented to exemplify the invention and are not to be considered as limiting the scope of the invention. Example 1 provides a non-limiting list of examples of compounds according to the invention. Example 2 illustrates the preparation of different compounds according to the invention. Example 3 illustrates *in vitro* anti-tumor effects of several compounds according to the invention.

### Examples

The practice of the present invention will employ, unless otherwise indicated, conventional techniques of synthetic organic chemistry, biological testing, and the like, which are within the skill of the art. Such techniques are explained fully in the literature.

### Example 1 Non-limitinq examples of compounds according to the invention having general formula IB are listed hereunder in Table A

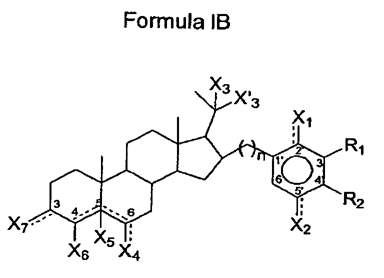

**TABLE A**

| **X**_{**1**} | **X**_{**2**} | **X**_{**3**} | **X**_{**3**}**'** | **X**_{**4**} | **X**_{**5**} | **X**_{**6**} | **X**_{**7**} | **R**_{**1**} | **R**_{**2**} | **n** |
|---|---|---|---|---|---|---|---|---|---|---|
| -OMe | -OMe | =O | | -H | -** | -H | β-D-glucopyranosyl | H | H | 0 |
| -OMe | -OMe | =O | | -H | -** | -H | galactopyranosyl | H | H | 0 |
| -OMe | -OMe | =O | | -H | -** | -H | mannopyranosyl | H | H | 0 |
| -OMe | -OMe | =O | | -H | -** | -H | xylopyranosyl | H | H | 0 |
| -OMe | -OMe | =O | | -H | -** | -H | cellobiosyl | H | H | 0 |
| -OMe | -OMe | =O | | -H | -** | -H | lactosyl | H | H | 0 |
| -OMe | -OMe | =O | | -H | -** | -H | glucofuranosyl | H | H | 0 |
| -OMe | -OMe | =O | | -H | -** | -H | maltosyl | H | H | 0 |
| -OMe | -OMe | =O | | -H | -** | -H | gentiobiosyl | H | H | 0 |
| -OMe | -OMe | =O | | galactopyranosyl | -** | -H | =O | H | H | 0 |
| -OMe | -OMe | =O | | mannopyranosyl | -** | -H | =O | H | H | 0 |
| -OMe | -OMe | =O | | xylopyranosyl | -** | -H | =O | H | H | 0 |
| -OMe | -OMe | =O | | cellobiosyl | -** | -H | =O | H | H | 0 |
| -OMe | -OMe | =O | | lactosyl | -** | -H | =O | H | H | 0 |
| -OMe | -OMe | =O | | glucofuranosyl | -** | -H | =O | H | H | 0 |
| -OMe | -OMe | =O | | maltosyl | -** | -H | =O | H | H | 0 |
| -OMe | -OMe | =O | | gentiobiosyl | -** | -H | =O | H | H | 0 |

| | | | | | | | | | | |
|---|---|---|---|---|---|---|---|---|---|---|
| ** refers to fact that X₅ participates to a double bond between the carbon atoms in position 5 and 6 | | | | | | | | | | |

### Example 2 Preparation of steroid compounds according to the invention

The present example provides evidence for the preparation of different compounds according to the invention, **UBS1664, UBS3268, UBS3270, UBS3285, UBS3327** and UBS3328. The prepared compounds and their intermediates are represented in Table B. In addition, this example also illustrates the preparation of a reference compound, UBS881. The compounds and their intermediate products are schematically represented in Table B.

### 1. Preparation of compound UBS1740

**UBS1697** was prepared by hydrogenating the compound having formula IV (100mg, 1.81 10⁻⁴mole) in 10ml of ethyl acetate (100mg of 10% Pd/C, H₂ at 45psi) for 24 hours. The palladium was filtered and the solvent was evaporated under reduced pressure. The obtained product, was purified by flash chromatography on silica gel (hexane/acetone 95/5) to give 77mg of compound **UBS1697.** The yield of this preparation process was 77%.

A solution of 2,5-dimethoxybenzene (391 mg, 1.80 10⁻³mole) and 1,2-dibromo-ethane (337mg, 1.79 10⁻³mole) in dry tetrahydrofuran (2ml) was added dropwise over 15min to a stirred mixture of Mg (200mg, 8.23 10⁻³mole) and I₂ (trace amount) in dry tetrahydrofuran (2ml) under N₂. After the addition, a solution of the compound UBS1697 (100mg, 1.90 10⁻⁴ mole) in dry tetrahydrofuran (1ml) was added dropwise over 5min. After 15min, a saturated NH₄Cl solution was added and the mixture was extracted with ether. The ether solution was washed with brine, dried (MgSO₄), filtered and concentrated in vacuo. The residue was chromatographed over silica gel (hexane/acetone 95/5) in order to provide 84mg of compound UBS1717. The yield of this preparation process was 67%.

Subsequently, a 1M solution of n-Bu4NF (200µl, 2 10⁻⁴ mole) was added to a solution of the compound (UBS1717) (50mg, 7.21 10⁻⁵ mole) in tetrahydrofuran (5ml) and the mixture was stirred for 3 days at room temperature. Purification of the crude mixture by silica gel flash chromatography (hexane/AcOEt 3/1) provided 25mg of the compound **UBS1740.** The yield of this preparation process was 76%.

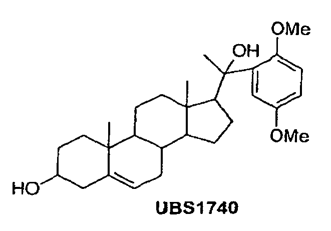

### 2. Preparation of compound UBS1664

In a similar manner as described for the preparation of UBS1717, the compound of formula IV (200mg, 3.62 10⁻⁴ mole) was treated with 2,5-dimethoxybenzene (314mg, 1.60 10⁻³mole) and magnesium (150mg, 5.78 10⁻³ mole) to obtain 60mg of the compound **UBS1513**. The yield of this preparation process was 24%.

In a similar manner as described as described for the preparation of UBS1740, the compound UBS1513 (150mg. 2.17 10-4 mole) was treated with a solution 1M of *n*-Bu₄NF (650µl, 6.52 10⁻⁴ mole) in tetrahydrofuran to give 700mg of compound **UBS1634**. The yield of this process was 70%.

PCC (238mg, 1.1 10⁻³ mole) was added in one portion to a solution of steroid II_{B1} (100mg, 2.20 10⁻⁴ mole) in dry CH₂Cl₂ (10ml) for 48h. Subsequent addition of Et₂O and filtration provided an organic solution, which was washed with water, dried, filtered and evaporated to give crude product. Purification of this crude mixture by silica gel chromatography (hexane/AcOEt 1/2) provided pure compound **UBS1664.** The yield of this process was 61%.

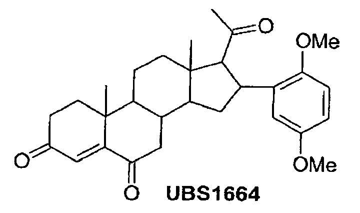

### 3. Preparation of compound UBS3268

UBS3267 was prepared by coupling at -20°C the compound UBS1634 (50mg, 0.11 10⁻³ mole) in 8ml of dichloromethane, 2ml of toluene and tetrabenzoylglucoside bromide (131 mg, 0.19 10⁻³ mole) in presence of silver trifluoromethane sulfonate (52mg, 0.19 10⁻³ mole) and allyltrimethylsilane (72mg, 0.62 10⁻³ mole). Tetrabenzoylglucoside bromide and others carbohydrate derivatives were prepared according to the procedure described in Steroids 63:44-49, 1998. The mixture was stirred overnight at room temperature. Purification of the crude mixture by silica gel chromatography (cyclohexane/AcOEt 8/2) provided 14mg of the compound UBS3267. The yield of this preparation process was 91%.

Subsequently, a solution of methanolate (0.084ml, 0.46 10⁻³ mole) in methanol was added at room temperature for 30 min to a stirred mixture of UBS3267 (80mg, 7.76 10⁻⁵ mole) in methanol. After neutralization and evaporation, the residue was purified by column chromatography on silica gel (CH₂Cl₂/MeOH 95/5) in order to provide 43mg of the compound **UBS3268**. The yield of this preparation process was 90%.

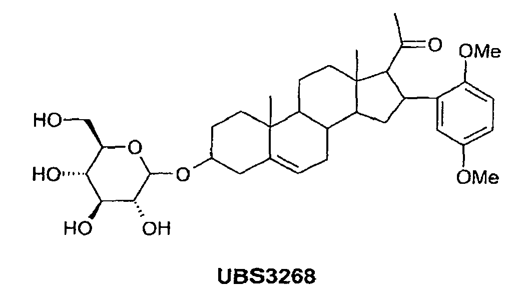

### 4. Preparation of compound UBS3270

In a similar manner as described for the preparation of UBS3267, the compound UBS1634 (60mg, 0.13 10⁻³ mole) was treated with tetrabenzoylmannoside bromide (158mg, 0.24 10⁻³ mole) in presence of silver trifluoromethane sulfonate (62mg, 0.24 10⁻³ mole) and allyltrimethylsilane (120µl, 0.74 10⁻³ mole) to obtain 112mg of the compound UBS3269. The yield of this preparation process was 82%.

In a similar manner as described for the preparation of UBS3268, the compound UBS3269 (80mg, 7.76 10⁻⁵ mole) was treated with methanolate (0.084ml, 0.46 10⁻³ mole) in methanol at room temperature for 30min to give 28mg of compound **UBS3270**. The yield of this preparation process was 58%.

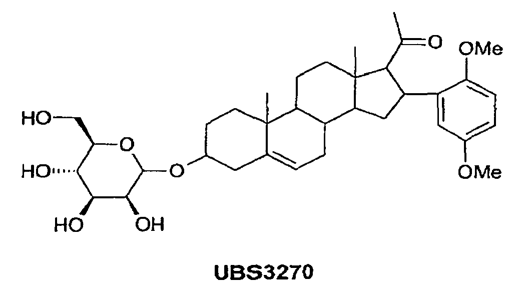

### 5. Preparation of compound UBS3327

In a similar manner as described for the preparation of UBS3267, the compound UBS1634 (50mg, 0.11 10⁻³ mole) was treated with octabenzoylcellobioside bromide (188mg, 0.16 10⁻³ mole) in presence of silver trifluoromethane sulfonate (44mg, 0.15 10⁻³ mole) and allyltrimethylsilane (72mg, 0.62 10⁻³ mole) to obtain 126mg of the compound of formula IB. The yield of this preparation process was 75%.

In a similar manner as described for the preparation of UBS3268, the later compound of formula IB (120mg, 7.9 10⁻⁵ mole) was treated with methanolate (0.143ml, 7.9 10⁻⁴ mole) in methanol at room temperature for 30min to give 73mg of compound **UBS3327**. The yield of this preparation process was 69%.

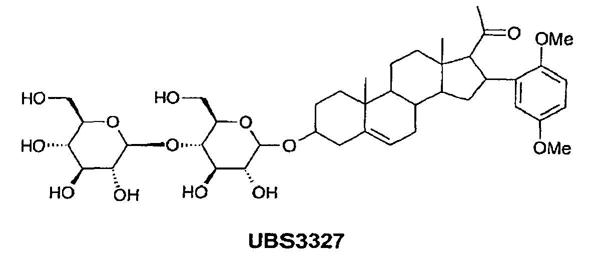

### 6. Preparation of compound UBS3328

In a similar manner as described for the preparation of UBS3267, the compound UBS1634 (50mg, 0.11 10⁻³ mole) was treated with octabenzoylgentiobioside bromide (188mg. 0.16 10⁻³ mole) in presence of silver trifluoromethane sulfonate (44mg, 0.15 10⁻³ mole) and allyltrimethylsilane (72mg, 0.62 10⁻³ mole) to obtain 57mg of the compound of formula IB. The yield of this preparation process was 34%.

In a similar manner as described for the preparation of UBS3268, the later compound of formula IB (45mg, 3.0 10⁻⁵ mole) was treated with methanolate (54µl, 3 10⁻⁴ mole) in methanol at room temperature for 30min to give 20mg of compound **UBS3328**. The yield of this preparation process was 86%.

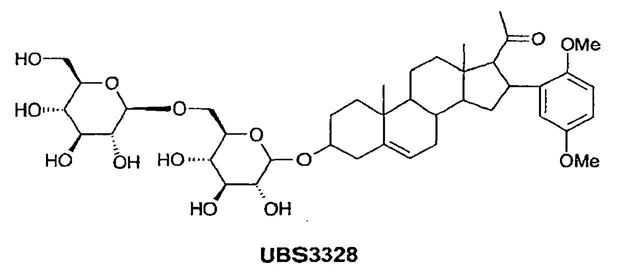

### 7. Preparation of compound UBS3285

A solution of tetrabenzylgalactopyranose (50mg, 9.2 10⁻⁵ mole), p-toluenesulfonyl chloride (20mg, 1 10⁻⁴ mole), tetrabutylammonium iodide (20mg, 5 10⁻⁵ mole) and the compound UBS1634 (150mg, 3 10⁻⁴ mole) in 10ml of dichloromethane is stirred with 40% aqueous NaOH (5ml) at room temperature. After 48h the organic layer is separated, washed with H₂O and dried (MgSO₄). The solvent is evaporated and the crude product is chromatographed on silica gel using (cyclohexane/AcOEt 9/1) in order to provide 25mg of compound having formula lB. The yield of this preparation process was 56%.

Subsequently, the later compound (20mg, 2 10⁻⁵ mole) in 5ml of ethanol and 5ml of AcOEt. Pd/C (20mg) and cyclohexene (1ml) was added and the mixture was heating under reflux for 2h. The palladium was filtered and the solvent was evaporated under reduced pressure to give 12mg of compound UBS3285. The yield of this preparation process was 99%.

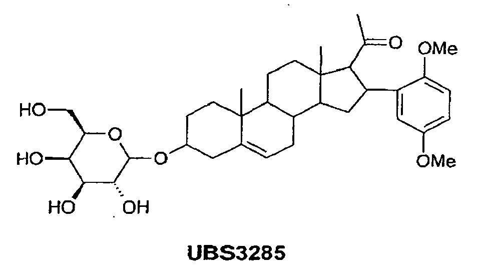

### 8. Preparation of compound UBS881

The compound UBS881 was obtained starting from cholesterol. In a similar manner as described for the preparation of UBS1664, cholesterol (400mg, 1.03 10⁻³ mole) was treated with PCC (1.550g, 7.21 10⁻³ mole) to obtain compound **UBS881**. This product is known to be isolated from *Cinachyrella voeltzkowi,* and shows an anti-cancer activity. It was used as reference compound in the below-described experiments

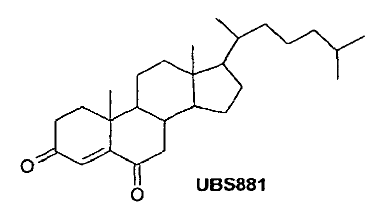

**TABLE B Compounds and their intermediates according to the invention**

| | **P** | **X**_{**1**} | **X**_{**2**} | **X**_{**3**} | **X**_{**3**}**'** | **X**_{**4**} | **X**_{**5**} | **X**_{**6**} | **X**_{**7**} | **R**_{**1**} | **R**_{**2**} | **n** |
|---|---|---|---|---|---|---|---|---|---|---|---|---|
| UBS1513 | tBuPh₂Si | -OMe | -OMe | =O | | -H | -** | -H | -O- | H | H | 0 |
| UBS1634 | - | -OMe | -OMe | =O | | -H | -** | -H | -OH | H | H | 0 |
| UBS1664 | - | -OMe | -OMe | =O | | =O | -* | -H | =O | H | H | 0 |
| UBS3267 | - | -OMe | -OMe | =O | | -H | -** | -H | Tetrabenzoyl D-Glucosyl | H | H | 0 |
| UBS3268 | - | -OMe | -OMe | =O | | -H | -** | -H | D-Glucosyl | H | H | 0 |
| UBS3269 | - | -OMe | -OMe | =O | | -H | -** | -H | Tetrabenzoyl D-Mannosyl | H | H | 0 |
| UBS3270 | - | -OMe | -OMe | =O | | -H | -** | -H | D-Mannosyl | H | H | 0 |
| UBS3285 | - | -OMe | -OMe | =O | | -H | -** | -H | D-Galactosyl | H | H | 0 |
| UBS3327 | - | -OMe | -OMe | =O | | -H | -** | -H | D-Collobiosyl | H | H | 0 |
| UBS3328 | - | -OMe | -OMe | =O | | -H | -** | -H | D-Gentiobiosyl | H | H | 0 |

| | | | | | | | | | | | | |
|---|---|---|---|---|---|---|---|---|---|---|---|---|
| * refers to fact that X₅ participates to a double bond between the carbon atoms in position 4 and 5 ** refers to fact that X₅ participates to a double bond between the carbon atoms in position 5 and 6 | | | | | | | | | | | | |

### Example 3 Effect of different compounds according to the invention on overall cell growth of a cell line

In order to characterize the *in vitro* activities of the compounds according to the invention, MTT tests were carried out. The MTT test, which is a well-known test in the art, is an indirect technique that rapidly measures, i.e. within 5 days, the effect of a given product on the overall cell growth. This test measures the number of metabolically active living cells that are able to transform the MTT product (3-(4,5-dimethylthiazol-2-yl)-2,5 diphenyl tetrazolium bromide), having a yellowish color, to the blue product formazan dye by mitochondrial reduction. The amount of formazan obtained at the end of the experiment is measured with a spectrophotometer and is directly proportional to the number of living cells. Determination of the optical density enables a quantitative measurement of the effect of the investigated compounds as compared to the control condition (untreated cells) and to compare it to other reference compound. In the following examples different compounds according to the invention were tested and compared to the reference compound being UBS881.

Six human cancer cell lines, described in Table C, were tested in the presence of the extract according to the invention. These cell lines represent four histological cancer types, being glioma cancer (cell line Hs683 and U-373 MG), colon cancer (cell ImeHCT-15 and LoVo), lung cancer (cell line A549) and bladder cancer (cell line J82). The cells were allowed to grow in 96-well micro wells with a flat bottom with an amount of 100 µl of cell suspension per well to have 1000 to 5000 celis/well depending on cell type. Each cell line was seeded in its own cell culture medium (Table C).

**TABLE C Human cancer cell lines and corresponding cell culture medium used for the MTT experiments**

| **Cell lines** | **ATCC code** | **Tissue** | **Medium** | **Literature Ref.** |
|---|---|---|---|---|
| **Hs683** | HTB-138 | Glioma | MEM 5% serum | J. Natl. Cancer Inst. 56: 843-849, 1976; *ibid. 58:* 1455-1463, 1977 |
| **U-373 MG** | HTB-17 | Glioma | MEM 5% serum | Acta Pathol. Microbial. Scand. *74:* 465-486, 1968 |
| **HCT-15** | CCL-225 | Colon | MEM 5% serum | Cancer Res. 39: 1020-1025, 1979 |
| **LoVo** | CCL-229 | Colon | MEM 5% serum | Exp. Cell Res. *101:* 414-416, 1976; J. Natl. Cancer Inst. *61:* 75-83, 1978; Cancer Res. *39:* 2630-2636, 1979 |
| **A549** | CCL-185 | Lung | MEM 5% serum | J. Natl. Cancer Inst. *51:* 1417-1423, 1973; Int. J. Cancer *17:* 62-70, 1976 |
| **J82** | HTB-1 | Bladder | MEM 5% serum | Br. J. Cancer *38:* 64-76, 1978; In Vitro models for cancer research Vol iV. CRC Press, 103-125, 1986 |

After a 24-hour period of incubation at 37°C, the culture medium is replaced by 100 µl of fresh medium in which the compound to be tested has been dissolved at different required concentrations. Different compounds were tested at 10⁻⁷ M, 5X10⁻⁷ M, 10⁻⁶ M, 5X10⁻⁶ M, 10⁻⁵ M, 5X10⁻⁵ M, 10⁻⁴ M, 5X10⁻⁴ M, and 10⁻³ M. Each experimental condition was carried out in hexaplicate. The compound tested was UBS1664. As a reference, UBS881 was used.

After 72 hours of incubation at 37°C with the compound (experimental conditions) or without the compound (control condition), the medium was replaced by 100 µl MTT at the concentration of 1 mg/ml dissolved in RPMI. The micro wells were subsequently incubated during 3 hours at 37° C and centrifuged at 400g during 10 minutes. The MTT was removed and formazan crystals formed, were dissolved in 100 µl DMSO. The micro wells were shaken for 5 minutes and read on a spectrophotometer at the wavelengths of 570 nm corresponding to the maximum formazan absorbance wavelength, and of 630 nm, which is the background noise wavelength.

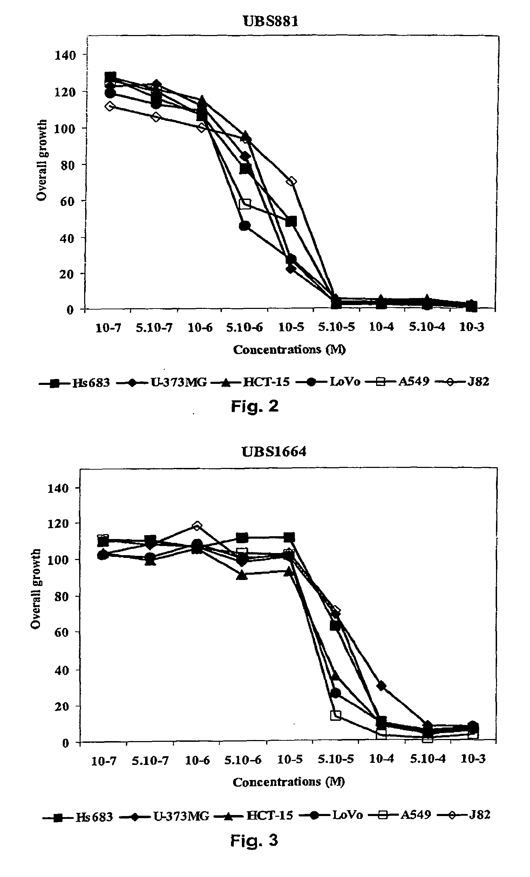

For each experimental condition, the mean OD associated with the SEM (standard error of the mean) for each condition (6 wells) was calculated. The percentage of remaining living cells in comparison with the control was calculated. Results of these experiments are represented in figures 2 to 3.

**Figure 2** represents the cytotoxic activity of the UBS881 on the 6 tested cancer cell lines. In **figure 3** it is shown that the compound UBS1664 induced cytotoxic activity on all 6 tested cell lines. The cytotoxic activity was stronger on HCT-15, LoVo and A549 lines than on Hs683, U-373MG and J82 cell lines. Thus, as illustrated on figures 2 to 3 the compounds according to the invention exerted an anti-tumor activity on different types of cancer cell lines.

The concentration at which the compounds according to the invention kill 50% of cell population, i.e. the IC₅₀ value, is represented in table D.

**TABLE D Comparison of the IC₅₀ value of compounds according to the invention**

| **compound** | **Hs683** | **U-373** | **HCT-15** | **LoVo** | **A549** | **J82** |
|---|---|---|---|---|---|---|
| **UBS881** | 10⁻⁵, 5.10⁻⁵ | 10⁻⁵, 5.10⁻⁶ | 10⁻⁵, 5.10⁻⁶ | 10⁻⁵, 5.10⁻⁵ | 10⁻⁵, 5.10⁻⁵ | 5.10⁻⁵, 10⁻⁵ |
| **UBS1664** | 10⁻⁴, 5.10⁻⁵ | 10⁻⁴, 5.10⁻⁵ | 5.10⁻⁵, 10⁻⁵ | 5.10⁻⁵, 10⁻⁵ | 5.10⁻⁵, 10⁻⁵ | 10⁻⁴, 5.10⁻⁵ |

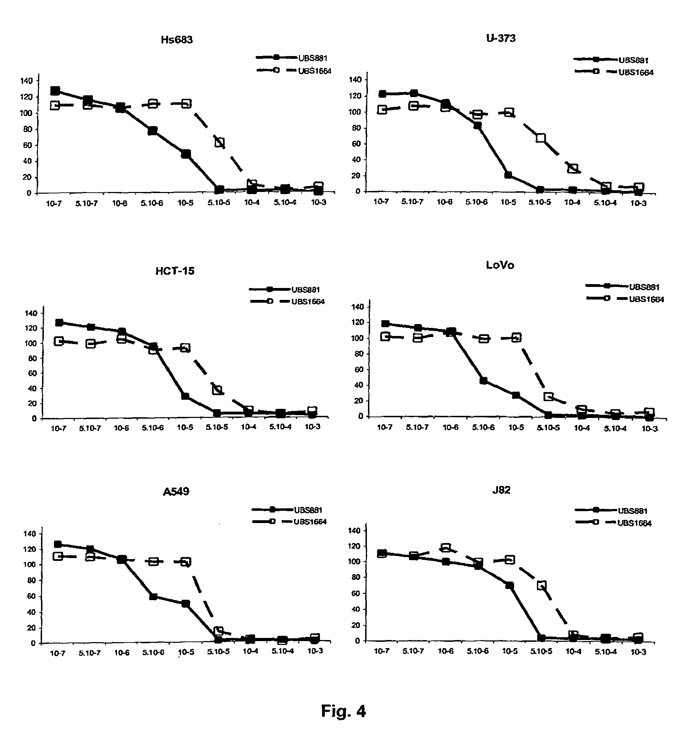

**Figure 4** compares the cytotoxic activity of UBS1664, UBS881 on 6 different cancer cell lines. The compound induced an anti-tumor effect on each tested cell line. The IC₅₀ values for UBS881, UBS1664, respectively ranged between [5.10⁻⁵, 5.10⁻⁶] and [10⁻⁴, 10⁻⁵].

In conclusion, the novel compound according to the invention tested exhibited an anti-tumor effect on the 6 human cancer cell lines assayed in the present experiments. These anti-tumor effects corresponded to marked decreases in the overall growth of these human cancers models belonging to four representative histological types.

### Example 4 Effect of compounds according to the invention on cell migration

The present example illustrates the effect of the compounds UBS881, UBS1664, UBS3285, UBS3327 and UBS3328 according to the invention on the migration of cancer cells.

Cells of different cancer lines, i.e. U-373 MG (Glioma cancer), Hs578T (breast cancer) and A549 (lung cancer) were seeded on culture flask 48 hours before the migration experiment. On the test day, cells were treated with or without compounds UBS881, UBS1664, UBS3285, UBS3327 and UBS3328 in closed Falcon dishes containing a buffered medium at a controlled temperature (37.0 ± 0.1°C) for 12 or 24 hours. The compounds were administered at two non-cytotoxic concentrations (10⁻⁶ M and 10⁻⁷ M) for UBS881 and UBS1664, and at 4 concentrations (10⁻⁷ M to 10⁻¹⁰ M) for UBS3285, UBS3327 and UBS3328. Migration of the cells was observed by means of a CCD-camera mounted on a phase-contrast microscope. A statistical analyse of the migration, with the non-parametric Mann-Whitney test, was established for 25% of the most motile cells and for the entire cell population for UBS881 and UBS1664, and established for 25% - 50% of the most motile cells and for the entire cell population for UBS3285, UBS3327 and UBS3328 compounds. The table E below illustrates the anti-migratory effect of the compound according to the invention.

**TABLE E Anti-migratory effect of the compounds UBS1664, UBS3285, UBS3327 and UBS3328 on cells of cancer cell lines**

| Compounds | Cell lines | Max. effects | Conditions |
|---|---|---|---|
| UBS881 | U-373 MG | -24% / p < 0,001 | For 24 hours on the 25% of most motile cells, at 10⁻⁷ M |
| UBS1664 | U-373 MG | -27% / p < 0,001 | For 24 hours on the 25% of most motile cells, at 10⁻⁷ M |
| | A549 | -15% / P < 0,05 | For 12 hours on the entire cell population, at 10⁻⁷ M |
| UBS3285 | U-373 MG | - 22% / p < 0.001 | For 24 hours on the 50% of most motile cells, at 10⁻⁸ M |
| UBS3327 | A549 | - 34% / p < 0.001 | For 24 hours on the 25% of most motile cells, at 10⁻⁸ M |
| | Hs578T | -21% / p < 0.001 | For 24 hours on the 25% of most motile cells, at 10⁻⁷ M |
| UBS3328 | A 549 | -40% / p< 0.001 | For 24 hours on the 25% of most motile cells, at 10⁻⁷ M |
| | U-373 MG | -27% / p< 0.001 | For 12 hours on the entire cell population, at 10⁻¹⁰ M |

In conclusion, the compounds UBS881, UBS1664, UBS3285, UBS3327 and UBS3328 induced a decrease in the migration level of U-373 MG, Hs578T and/or A549 cancer cells at the weak studied concentrations.

## Claims

1. A compound having the structural formula IB or pharmaceutically acceptable salts, stereoisomeric forms, or racemic mixtures thereof, 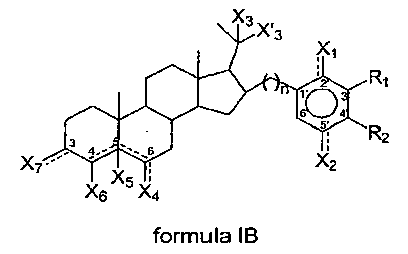 wherein X₁ and X₂ are -OMe,
wherein R₁ and R₂ are -H,
wherein X₃ participates together with X₃' to an oxo functional group,
wherein X₆ is hydrogen,
wherein n is 0,
wherein X₅ participates to a double bond between the carbon atoms in position 5 and 6 or to a double bond between the carbon atoms in position 4 and 5, and
wherein X₄ and X₇ are independently selected from the group comprising hydrogen, oxo, hydroxyl, glucosyl, fructosyl, galactosyl, mannosyl, ribosyl, ribulosyl, xylulosyl, erythrosyl, erythrulosyl, rhamnosyl, threosyl, sorbosyl, tagatosyl, fucosyl, arabinosyl, xylofuranosyl, lyxosyl, talosyl, psicosyl, idosyl, gulosyl, altrosyl, allosyl, mannoheptulosyl, sedoheptulosyl, abequosyl, isomaltosyl, kojibiosyl, laminarabiosyl, nigerosyl, primeverosyl, rutinosyl, tyvelosyl, maltosyl, lactosyl, sucrosyl, cellobiosyl, trehalosyl, gentiobiosyl, melibiosyl, turanosyl, sophorosyl, isosucrosyl, raffinosyl, gentianosyl, 2-amino-2-deoxy glucosyl, 2-acetamido-2-deoxy-glucosyl, 2-amino-2-deoxy galactosyl, 2-acetamido-2-deoxy-galactosyl, 2-amino-2-deoxy mannosyl, 2-acetamido-2-deoxy-mannosyl, 2-amino-1,3-cyclohexanediol, L or D isomers thereof, α or β form thereof, pyranose or furanose form thereof, combination thereof, deoxy derivatives thereof, hydroxyl-protected acetate derivatives thereof, disaccharide thereof, trisaccharide thereof, oligosaccharide and polysaccharide thereof.

2. A compound according to claim 1,
wherein X₁, X₂, R₁, R₂, X₃, X₃', X₆ and n are as defined in claim 1,
wherein X₅ participates to a double bond between the carbon atoms in position 5 and 6, and
wherein X₄ and X₇ are independently selected from the group comprising hydrogen hydroxyl, glucosyl, fructosyl, galactosyl, mannosyl, fucosyl, cellobiosyl, gentiobiosyl, lactosyl, maltosyl, xylopyranosyl, 2-amino-2-deoxy glucosyl, 2-acetamido-2-deoxy-glucosyl, 2-amino-2-deoxy galactosyl, 2-acetamido-2-deoxy-galactosyl, L or D isomers thereof, α or β form thereof, pyranose or furanose form thereof, combination thereof, deoxy derivatives thereof, hydroxyl-protected acetate derivatives thereof, disaccharide or trisaccharide thereof, oligosaccharide and polysaccharide thereof.

3. A compound according to claim 1 or 2, wherein said compound is designated as compound UBS3327.

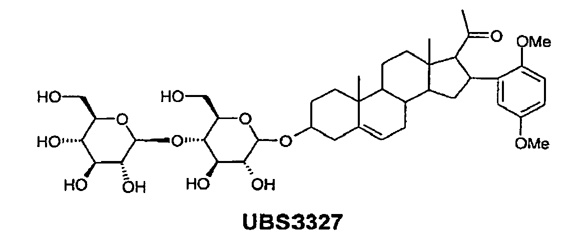

4. A compound according to claim 1 or 2, wherein said compound is designated as compound UBS3328.

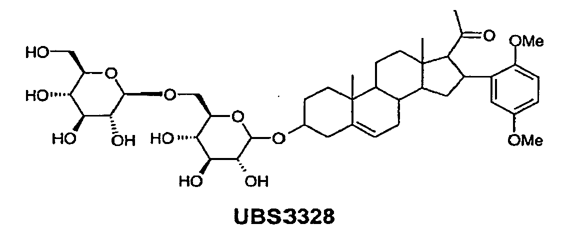

5. A compound according to claim 1 or 2, wherein said compound is designated as compound UBS 3268.

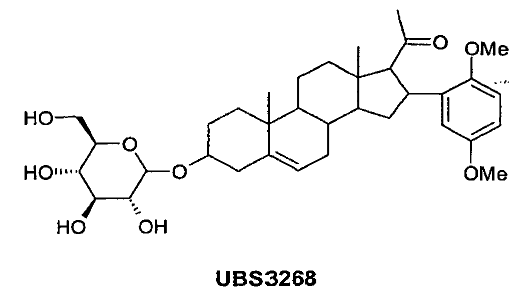

6. A compound according to claim 1 or 2, wherein said compound is designated as compound UBS 3270.

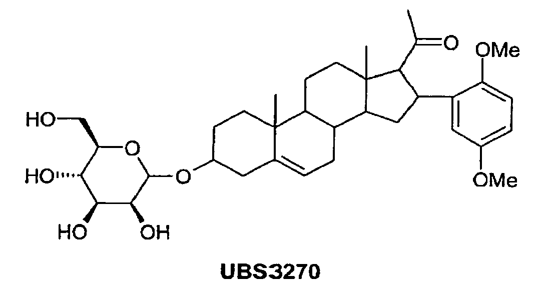

7. A compound according to claim 1 or 2, wherein said compound is designated as compound UBS 3285.

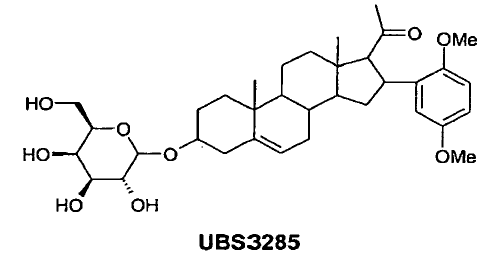

8. A compound according to claim 1 or 2, wherein X₁ and X₂ are -OMe, wherein R₁ and R₂ are -H, wherein X₃ participates together with X₃' to an oxo functional group, wherein X₆ is hydrogen, wherein n is 0, and wherein X₅ participates to a double bond between the carbon atoms in position 5 and 6, wherein X₄ is hydrogen, and wherein X₇ is lactosyl.

9. A compound according to claim 1 or 2, wherein X₁ and X₂ are -OMe, wherein R₁ and R₂ are -H, wherein X₃ participates together with X₃' to an oxo functional group, wherein X₆ is hydrogen, wherein n is 0, and wherein X₅ participates to a double bond between the carbon atoms in position 5 and 6, wherein X₄ is hydrogen, and wherein X₇ is maltosyl.

10. A compound according claim 1 or 2, wherein X₁ and X₂ are -OMe, wherein R₁ and R₂ are -H, wherein X₃ participates together with X₃' to an oxo functional group, wherein X₆ is hydrogen, wherein n is 0, and wherein X₅ participates to a double bond between the carbon atoms in position 5 and 6, wherein X₄ is hydrogen, and wherein X₇ is hydroxyl.

11. Compound according to claim 1, wherein X₁, X₂, R₁, R₂, X₃, X₃', X₆ and n are as defined in claim 1,
wherein X₅ participates to a double bond between the carbon atoms in position 4 and 5, and
wherein X₄ and X₇ are independently selected from the group comprising oxo, glucosyl, galactosyl, mannosyl, xylofuranosyl, maltosyl, lactosyl, cellobiosyl, gentiobiosyl, L or D isomers thereof, α or β form thereof, pyranose or furanose form thereof, combination thereof, deoxy derivatives thereof, hydroxyl-protected acetate derivatives thereof.

12. Compound according to claim 1 or 11, wherein said compound is designated as compound UBS1664

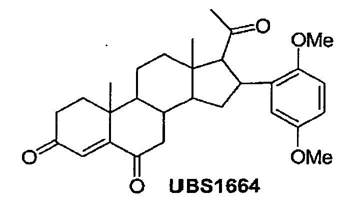

13. Method for synthesizing a compound having the structural formula IB 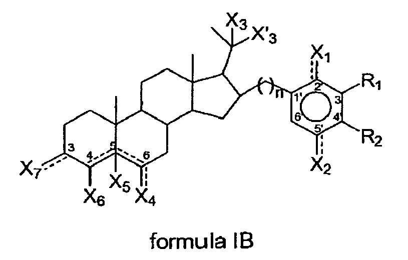 wherein X₁, X₂, X₃, X₄, X₅, X₆, X₇, R₁, R₂ and n are selected from the group as indicated in any of claims 1 to 12, said method comprising the steps of
a) providing a starting material having the structural formula IV, 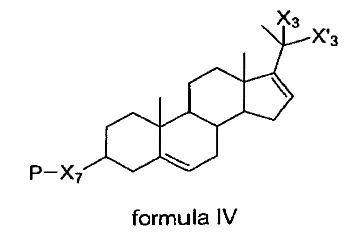 wherein X₃, X₃' and X₇ are selected from the group as indicated in any of claims 1 to 12, and wherein P is a protecting group,
b) effecting reaction between the compound of step a) with an organometallic compound having the structural formula V 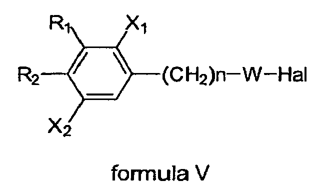 wherein X₁, X₂, R₁, R₂ and n are selected from the group as indicated in any of claims 1 to 12, wherein W is a metal or a combination of metals and wherein Hal is a halogen atom,
to result in an intermediate having the structural formula III'B 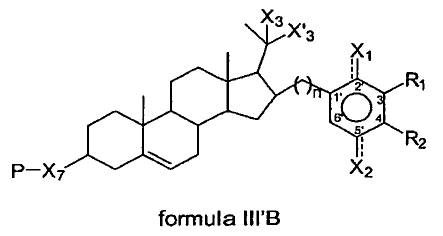 wherein X₁, X₂, X₃, X₃', X₇, R₁, R₂ and n are selected from the group as indicated in any of claims 1 to 12, and wherein p is a protecting group,
c) effecting reaction between the compound of step b) with an organometallic compound having the structural formula VI
Hal-W-X'₃ formula VI
wherein X'₃ is selected from the group as indicated in any of claims 1 to 12, wherein W is a metal or a combination of metals, and wherein Hal is a halogen atom,
to result in an intermediate having the structural formula IIIB 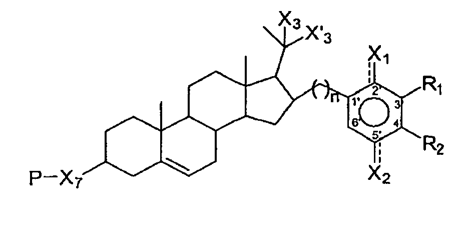 wherein X₁, X₂, X₃, X₃', X₇, R₁, R₂ and n are selected from the group as indicated in any of claims 1 to 12, wherein P is a protecting group,
d) deprotecting the X₇ group of the compound obtained in step c) to form an compound having the structural formula IIB 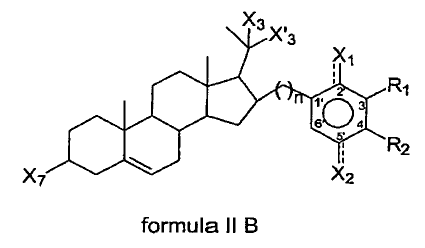 wherein X₁, X₂, X₃, X₃', X₇, R₁. R₂ and n are selected from the group as indicated in any of claims 1 to 12, and
e) oxidizing by reaction with a suitable oxidizing agent or agents to form a compound of formula IB
or
e) coupling an O-protected glycosyl or non-protected glycosyl to form a compound of formula IIB wherein X₁, X₂, X₃, X'₃, X₇, R₁, R₂ and n are selected from the group as indicated in any of claims 1 to 12 and X₇ is an O-protected glycosyl or a non-protected glycosyl, and
f) deprotecting the O-protected groups of glycosyl to form a compound of formula IB wherein X₁, X₂, X₃, X'₃, X₄, X₅, X₆, X₇. R₁, R₂ and n are selected from the group as indicated in any of claims 1 to 12.

14. A compound according to any of claims 1 to 12 for use as a medicament.

15. Use of a compound according to any of claims 1 to 12 for the preparation of a medicament for treating cancer.

16. A pharmaceutical composition comprising a pharmaceutically acceptable excipient and a therapeutically effective amount of a compound according to any of claims 1 to 12.

## Patentansprüche

1. Eine Verbindung, die die Struktur-Formel IB hat oder pharmazeutisch verträgliche Salze, stereoisomere Formen oder razemische Gemische davon, 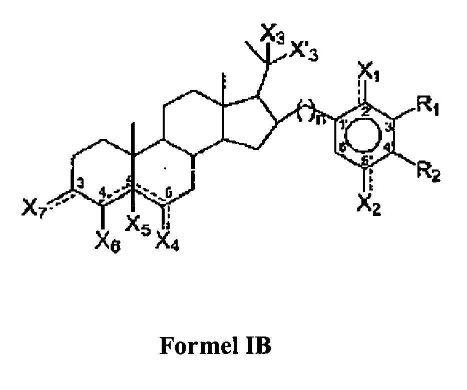 wobei X₁ und X₂ -OMe sind,
wobei R₁ und R₂ -H sind,
wobei sich X₃ zusammen mit X'₃ an einer Oxo-funktionellen Gruppe beteiligt,
wobei X₆ Wasserstoff ist,
wobei n 0 ist,
wobei sich X₅ an einer Doppelbindung zwischen den Kohlenstoffatomen der Position 5 und 6 oder an einer Doppelbindung zwischen den Kohlenstoffatomen der Position 4 und 5 beteiligt, und
wobei X₄ und X₇ unabhängig voneinander aus der Gruppe ausgewählt werden, bestehend aus Wasserstoff, Oxo, Hydroxyl, Glucosyl, Fructosyl, Galactosyl, Mannosyl, Ribosyl, Ribolosyl, Xylulosyl, Erythrosyl, Erythrulosyl, Rhamnosyl, Threosyl, Sorbosyl, Tagatosyl, Fucosyl, Arabinosyl, Xylofuranosyl, Lyxosyl, Talosyl, Psicosyl, Idosyl, Gulosyl, Altrosyl, Allosyl, Mannoheptulosyl, Sedoheptulosyl, Abequosyl, Isomaltosyl, Kojibiosyl, Laminarabiosyl, Nigerosyl, Primeverosyl, Rutinosyl, Tyvelosyl, Maltosyl, Lactosyl, Sucrosyl, Cellobiosyl, Trehalosyl, Gentiobiosyl, Melibiosyl, Turanosyl, Sophorosyl, Isosucrosyl, Raffinosyl, Gentianosyl, 2-Amino-2-desoxy-Glucosyl, 2-Acetamido-2-desoxy-glucosyl, 2-Amino-2-desoxy-galactosyl, 2-Acetamido-2-desoxy-galactosyl, 2-Amino-2-desoxy-mannosyl, 2-Acetamido-2-desoxy-mannosyl, 2-Amino-1,3-cyclohexandiol, L- oder D-Isomere davon, α oder β-Form davon, Pyranose- oder Furanoseform davon, eine Kombination davon, Desoxiderivate davon, Hydroxyl-geschützte Acetatderivate davon, ein Disaccharid davon, ein Trisaccharid davon, ein Oligosaccharid und Polysaccharid davon.

2. Eine Verbindung nach Anspruch 1,
wobei X₁, X₂, R₁, R₂, X₃, X'₃, X₆ und n wie in Anspruch 1 definiert sind,
wobei sich X₅ an eine Doppelbindung zwischen dem Kohlenstoffatomen der Position 5 und 6 beteiligt, und
wobei X₄ und X₇ unabhängig aus der Gruppe ausgewählt werden, bestehend aus Wasserstoff, Hydroxyl, Glucosyl, Fructosyl, Galactosyl, Mannosyl, Frucosyl, Cellobiosyl, Gentiobiosyl, Lactosyl, Maltosyl, Xylopyranosyl, 2-Amino-2-desoxy-glucosyl, 2-Acetamido-2-desoxy-glucosyl, 2-Amino-2-desoxy-galactosyl, 2-Acetamido-2-desoxy-galactosyl, L- oder D-Isomere davon, α- oder β-Form davon, Pyranose- oder Furanoseform davon, eine Kombination davon, Desoxyderivate davon, Hydroxyl-geschützte Acetatderivate davon, ein Disaccharid oder Trisaccharid davon, ein Oligosaccharid und Polysaccharid davon.

3. Eine Verbindung nach Anspruch 1 oder 2, wobei die Verbindung als Verbindung UBS3327 bezeichnet wird.

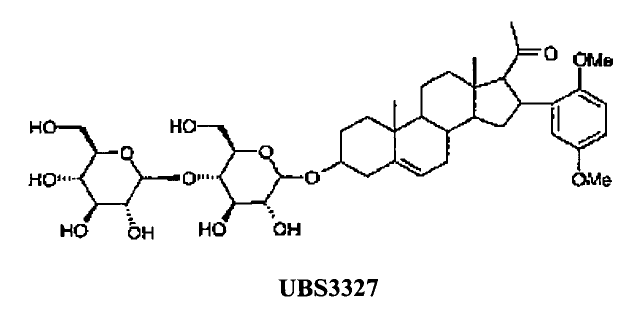

4. Eine Verbindung nach Anspruch 1 oder 2, wobei die Verbindung als Verbindung UBS3328 bezeichnet wird.

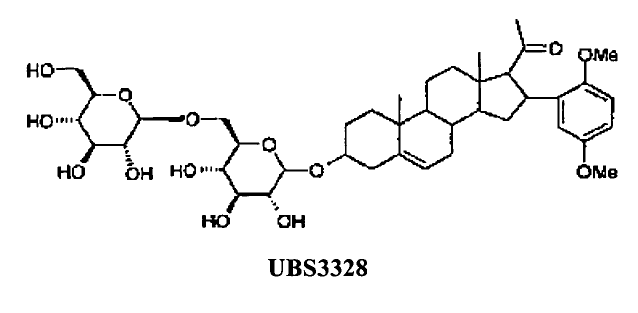

5. Eine Verbindung nach Anspruch 1 oder 2, wobei die Verbindung als Verbindung UBS3268 bezeichnet wird.

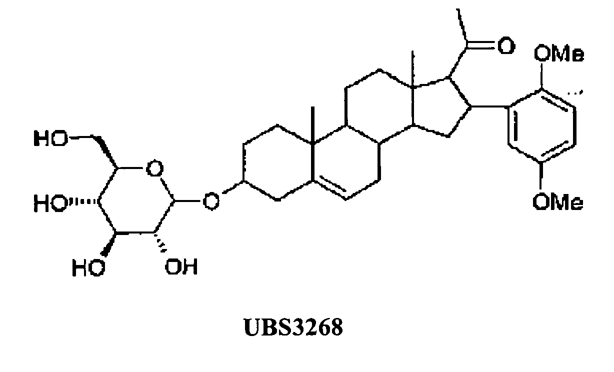

6. Eine Verbindung nach Anspruch 1 oder 2, wobei die Verbindung als Verbindung UBS3270 bezeichnet wird.

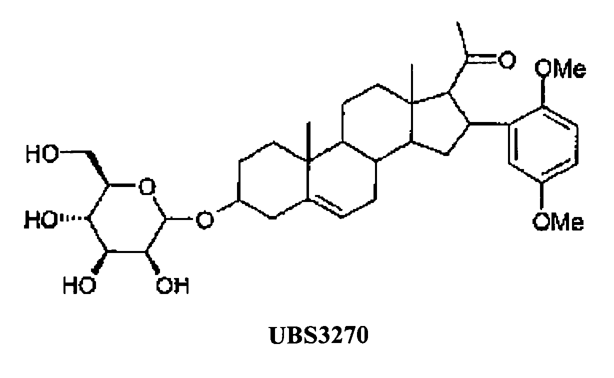

7. Eine Verbindung nach Anspruch 1 oder 2, wobei die Verbindung als Verbindung UBS3285 bezeichnet wird.

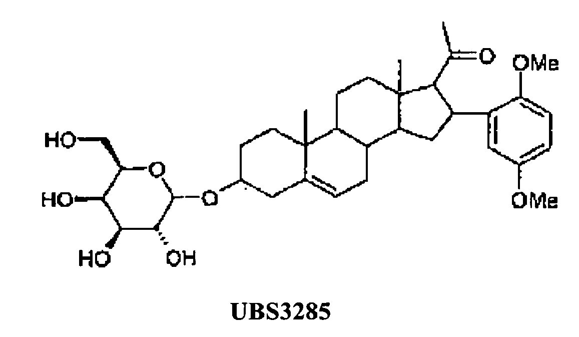

8. Eine Verbindung nach Anspruch 1 oder 2, wobei X₁ und X₂ -OMe sind, wobei R₁ und R₂ -H sind, wobei sich X₃ zusammen mit X'₃ an eine Oxo-funktionelle Gruppe beteiligt, wobei X₆ Wasserstoff ist, wobei n 0 ist, und wobei sich X₅ an eine Doppelbindung zwischen den Kohlenstoffatomen der Position 5 und 6 beteiligt, wobei X₄ Wasserstoff ist, und wobei X₇ Lactosyl ist.

9. Eine Verbindung nach Anspruch 1 oder 2, wobei X₁ und X₂ -OMe sind, wobei R₁ und R₂ -H sind, wobei sich X₃ zusammen mit X'₃ an eine Oxo-funktionelle Gruppe beteiligt, wobei X₆ Wasserstoff ist, wobei n 0 ist, und wobei sich X₅ an eine Doppelbindung zwischen den Kohlenstoffatomen der Position 5 und 6 beteiligt, wobei X₄ Wasserstoff ist, und wobei X₇ Maltosyl ist.

10. Eine Verbindung nach Anspruch 1 oder 2, wobei X₁ und X₂ -OMe sind, wobei R₁ und R₂ -H sind, wobei sich X₃ zusammen mit X'₃ an eine Oxo-funktionelle Gruppe beteiligt, wobei X₆ Wasserstoff ist, wobei n 0 ist, und wobei sich X₅ an eine Doppelbindung zwischen den Kohlenstoffatomen der Position 5 und 6 beteiligt, wobei X₄ Wasserstoff ist, und wobei X₇ Hydroxyl ist.

11. Verbindung nach Anspruch 1, wobei X₁, X₂, R₁, R₂, X₃, X'₃, X₆ und n wie in Anspruch 1 definiert sind,
wobei sich X₅ an eine Doppelbindung zwischen den Kohlenstoffatomen der Position 4 und 5 beteiligt, und
wobei X₄ und X₇ unabhängig aus der Gruppe ausgewählt werden, bestehend aus Oxo, Glucosyl, Galactosyl, Mannosyl, Xylofuranosyl, Maltosyl, Lactosyl, Cellobiosyl, Gentiobiosyl, L- oder D-Isomere davon, α- oder β-Form davon, Pyranose- oder Furanoseform davon, eine Kombination davon, Desoxyderivate davon, Hydroxyl-geschützte Acetatderivate davon.

12. Verbindung nach Anspruch 1 oder 11, wobei die Verbindung als Verbindung UBS1664 bezeichnet wird.

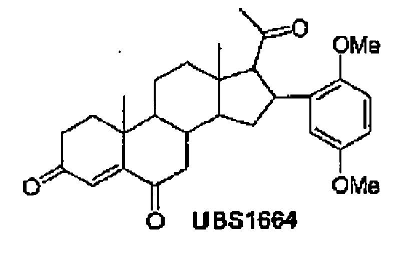

13. Verfahren für die Synthese einer Verbindung, die die Struktur-Formel IB hat, 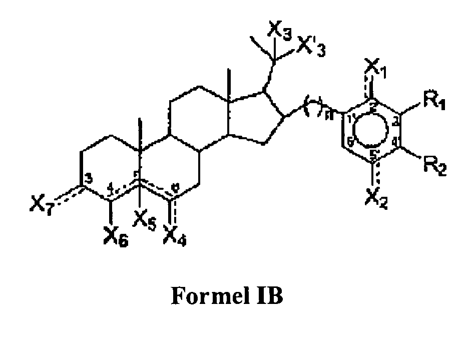 wobei X₁, X₂, X₃, X₄, X₅, X₆, X₇, R₁, R₂ und n aus der Gruppe ausgewählt werden, wie in einem der Ansprüche 1 bis 12 angezeigt, wobei das Verfahren die Schritte umfasst:
a) Zur-Verfügung-Stellen eines Ausgangsmaterials, das die Struktur-Formel IV hat, 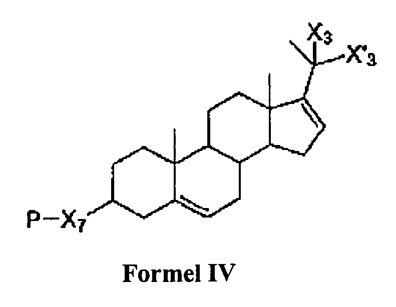 wobei X₃, X'₃ und X₇ aus der Gruppe ausgewählt werden, wie in einem der Ansprüche 1 bis 12 angezeigt, und wobei P eine Schutz-Gruppe ist,
b) Herbeiführen der Reaktion zwischen der Verbindung aus Schritt a) mit einer organometallischen Verbindung, die die Struktur-Formel V hat, 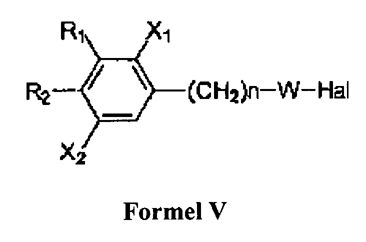 wobei X₁, X₂, R₁, R₂ und n aus der Gruppe ausgewählt werden, wie in einem der Ansprüche 1 bis 12 angezeigt, wobei W ein Metall oder eine Kombination aus Metallen ist, und wobei Hal ein Halogenatom ist,
so dass ein Zwischenprodukt resultiert, das die Struktur-Formel III'B hat, 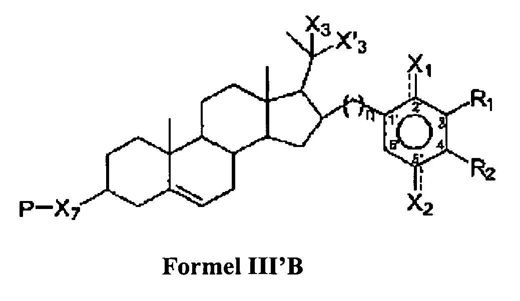 wobei X₁, X₂, X₃, X'₃, X₇, R₁, R₂ und n aus der Gruppe ausgewählt werden, wie in einem der Ansprüche 1 bis 12 angezeigt, und wobei P eine Schutz-Gruppe ist,
c) Herbeiführen der Reaktion zwischen der Verbindung aus Schritt b) mit einer organometallischen Verbindung, die die Struktur-Formel VI hat,
**Hal-W-X'**_{**3**} **Formel VI**
wobei X'₃ aus der Gruppe ausgewählt ist, wie in einem der Ansprüche 1 bis 12 angezeigt, wobei W ein Metall oder eine Kombination aus Metallen ist, und wobei Hal ein Halogenatom ist,
so dass ein Zwischenprodukt resultiert, das die Struktur-Formel IIIB hat, 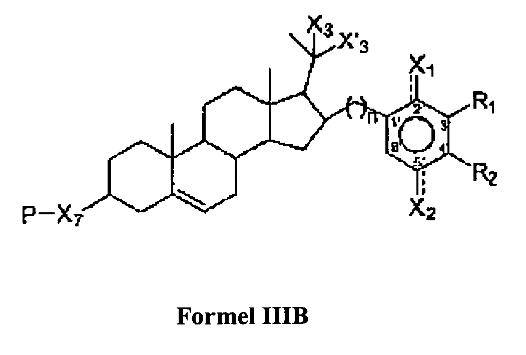 wobei X₁, X₂, X₃, X'₃, X₇, R₁, R₂ und n aus der Gruppe ausgewählt werden, wie in einem der Ansprüche 1 bis 12 angezeigt, wobei P eine Schutz-Gruppe ist,
d) Entfernen der X₇-Schutz-Gruppe aus der Verbindung, die in Schritt c) erhalten wurde, so dass eine Verbindung gebildet wird, die die Struktur-Formel IIB hat, 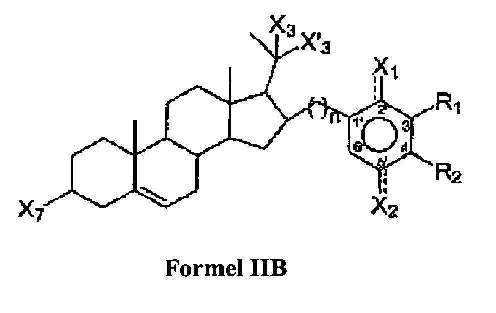 wobei X₁, X₂, X₃, X'₃, X₇, R₁, R₂ und n aus der Gruppe ausgewählt werden, wie in einem der Ansprüche 1 bis 12 angezeigt, und
e) Oxidieren durch eine Reaktion mit einem oder mehreren geeigneten Oxidationsmitteln, so dass eine Verbindung der Formel IB gebildet wird, oder
e) Koppeln eines O-geschützten Glycosyls oder nicht-geschützten Glycosyls, so dass eine Verbindung der Formel IIB gebildet wird, wobei X₁, X₂, X₃, X'₃, X₇, R₁, R₂ und n aus der Gruppe ausgewählt werden, wie in einem der Ansprüche 1 bis 12 angezeigt, und X₇ ein O-geschütztes Glycosyl oder ein nicht-geschütztes Glycosyl ist, und
f) Entfernen der O-Schutz-Gruppen aus dem Glycosyl, so dass eine Verbindung der Formel IB gebildet wird, wobei X₁, X₂, X₃, X'₃, X₄, X₅, X₆, X₇, R₁, R₂ und n aus der Gruppe ausgewählt werden, wie in einem der Ansprüche 1 bis 12 angezeigt.

14. Eine Verbindung nach einem der Ansprüche 1 bis 12 zur Verwendung als ein Medikament.

15. Verwendung einer Verbindung nach einem der Ansprüche 1 bis 12 für die Zubereitung eines Medikaments zur Behandlung von Krebs.

16. Eine pharmazeutische Zusammensetzung, die einen pharmazeutisch verträglichen Exzipienten und eine therapeutisch wirksame Menge einer Verbindung nach einem der Ansprüche 1 bis 12 umfasst.

## Revendications

1. Composé ayant la formule structurelle IB ou des sels acceptables pharmaceutiquement, des formes stéréo-isomères, ou des mélanges racémiques de ceux-ci, 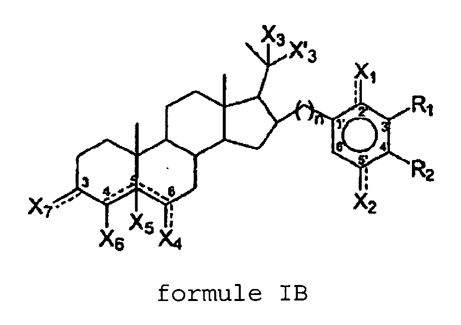 dans lequel X₁ et X₂ sont -OMe,
dans lequel R₁ et R₂ sont -H
dans lequel X₃ participe avec X₃' à un groupe fonctionnel oxo,
dans lequel X₆ est un hydrogène,
dans lequel n est 0,
dans lequel X₅ participe à une double liaison entre les atomes de carbone en position 5 et 6 ou à une double liaison entre les atomes de carbone en position 4 et 5, et
dans lequel X₄ et X₇ sont sélectionnés indépendamment parmi le groupe comprenant l'hydrogène, l'oxo, l'hydroxyle, le glucosyl, le fructosyl, le galactosyl, le mannosyl, le ribosyl, le ribulosyl, le xylulosyl, l'erythrosyl, l'erythrulosyl, le rhamnosyl, le threosyl, le sorbosyl, le tagatosyl, le fucosyl, l'arabinosyl, le xylofuranosyl, le lyxosyl, le talosyl, le psicosyl, l'idosyl, le gulosyl, l'altrosyl, l'allosyl, le mannoheptulosyl, le sedoheptulosyl, l'abequosyl, l'isomaltosyl, le kojibiosyl, le laminarabiosyl, le nigerosyl, le primeverosyl, le rutinosyl, le tyvelosyl, le maltosyl, le lactosyl, le sucrosyl, le cellobiosyl, le trehalosyl, le gentiobiosyl, le melibiosyl, le turanosyl, le sophorosyl, l'isosucrosyl, le raffinosyl, le gentianosyl, le 2-amino-2-déoxy glucosyl, le 2-acétamido-2-déoxy-glucosyl, le 2-amino-2-déoxy galactosyl, le 2-acétamido-2-déoxy-galactosyl, le 2-amino-2-déoxy mannosyl, le 2-acétamido-2-déoxy-mannosyl, le 2-amino-1,3-cyclohexanediol, les isomères L ou D de ceux-ci, la forme α ou β de ceux-ci, la forme pyranose ou furanose de ceux-ci, une combinaison de ceux-ci, des dérivés déoxy de ceux-ci, des dérivés acétate de ceux-ci dont l'hydroxyle est protégé, un disaccharide de ceux-ci, un trisaccharide de ceux-ci, un oligosaccharide et un polysaccharide de ceux-ci.

2. Composé selon la revendication 1,
dans lequel X₁, X₂, R₁, R₂, X₃, X₃', X₆ et n sont tels que définis dans la revendication 1,
dans lequel X₅ participe à une double liaison entre les atomes de carbone en position 5 et 6, et
dans lequel X₄ et X₇ sont sélectionnés indépendamment parmi le groupe comprenant l'hydrogène l'hydroxyle, le glucosyl, le fructosyl, le galactosyl, le mannosyl, le fucosyl, le cellobiosyl, le gentiobiosyl, le lactosyl, le maltosyl, le xylopyranosyl, le 2-amino-2-déoxy glucosyl, le 2-acétamido-2-déoxy-glucosyl, le 2-amino-2-deoxy galactosyl, le 2-acétamido-2-déoxy-galactosyl, les isomères L ou D de ceux-ci, la forme α ou β de ceux-ci, la forme pyranose ou furanose de ceux-ci, une combinaison de ceux-ci, les dérivés déoxy de ceux-ci, les dérivés acétate de ceux-ci dont l'hydroxyle est protégé, le disaccharide ou le trisaccharide de ceux-ci, l'oligosaccharide et le polysaccharide de ceux-ci.

3. Composé selon la revendication 1 ou 2, dans lequel ledit composé est conçu tel que le composé UBS3327.

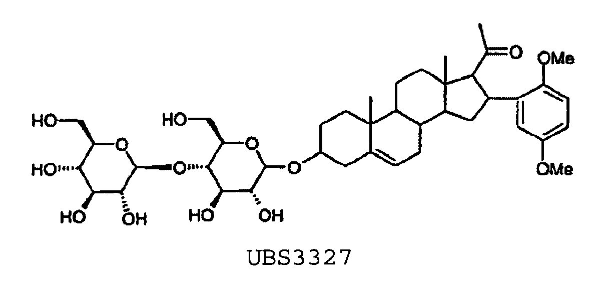

4. Composé selon la revendication 1 ou 2, dans lequel ledit composé est désigné tel que le composé UBS3328.

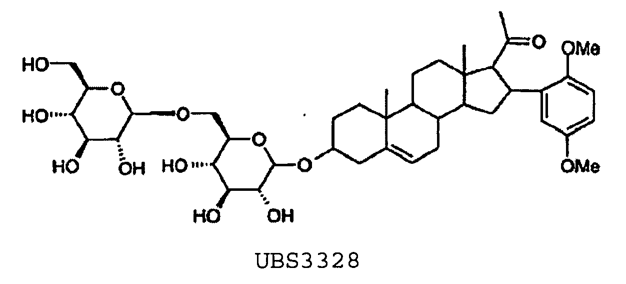

5. Composé selon la revendication 1 ou 2, dans lequel ledit composé est désigné tel que le composé UBS 3268.

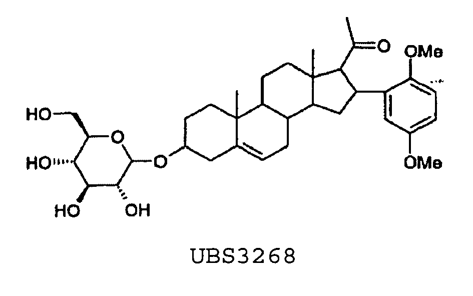

6. Composé selon la revendication 1 ou 2, dans lequel ledit composé est désigné tel que le composé UBS 3270.

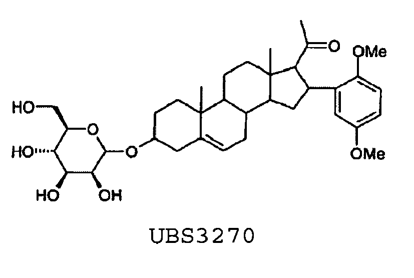

7. Composé selon la revendication 1 ou 2, dans lequel ledit composé est désigné tel que le composé UBS 3285.

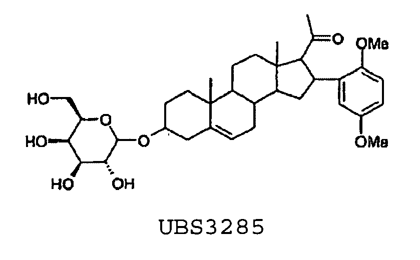

8. Composé selon la revendication 1 ou 2, dans lequel X₁ et X₂ sont -OMe, dans lequel R₁ et R₂ sont -H, dans lequel X₃ participe avec X₃' à un groupe fonctionnel oxo, dans lequel X₆ est un hydrogène, dans lequel n est 0, et dans lequel X₅ participe à une double liaison entre les atomes de carbone en position 5 et 6, dans lequel X₄ est un hydrogène, et dans lequel X₇ est un lactosyl.

9. Composé selon la revendication 1 ou 2, dans lequel X₁ et X₂ sont -OMe, dans lequel R₁ et R₂ sont -H, dans lequel X₃ participe avec X₃' à un groupe fonctionnel oxo, dans lequel X₆ est un hydrogène, dans lequel n est 0, et dans lequel X₅ participe à une double liaison entre les atomes de carbone en position 5 et 6, dans lequel X₄ est un hydrogène, et dans lequel X₇ est un maltosyl.

10. Composé selon la revendication 1 ou 2, dans lequel X₁ et X₂ sont -OMe, dans lequel R₁ et R₂ sont -H, dans lequel X₃ participe avec X₃' à un groupe fonctionnel oxo, dans lequel X₆ est un hydrogène, dans lequel n est 0, et dans lequel X₅ participe à une double liaison entre les atomes de carbone en position 5 et 6, dans lequel X₄ est un hydrogène, et dans lequel X₇ est un hydroxyle.

11. Composé selon la revendication 1, dans lequel X₁, X₂, R₁, R₂, X₃, X₃', X₆ et n sont tels que définis dans la revendication 1,
dans lequel X₅ participe à une double liaison entre les atomes de carbone en position 4 et 5, et
dans lequel X₄ et X₇ sont indépendamment sélectionnés parmi le groupe comprenant l'oxo, le glucosyl, le galactosyl, le mannosyl, le xylofuranosyl, le maltosyl, le lactosyl, le cellobiosyl, le gentiobiosyl, les isomères L ou D de ceux-ci, la forme α ou β de ceux-ci, la forme pyranose ou furanose de ceux-ci, une combinaison de ceux-ci, les dérivés déoxy de ceux-ci, les dérivés acétate de ceux-ci dont l'hydroxyle est protégé.

12. Composé selon la revendication 1 ou 11, dans lequel ledit composé est désigné tel que le composé UBS1664

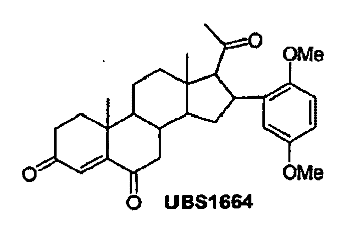

13. Procédé pour la synthèse d'un composé ayant la formule structurelle IB 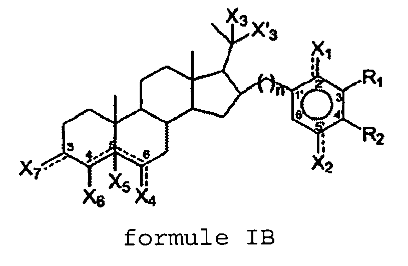 dans lequel X₁, X₂, X₃, X₄, X₅, X₆, X₇, R₁, R₂ et n sont sélectionnés parmi le groupe tel qu'indiqué dans l'une quelconque des revendications 1 à 12, ladite méthode comprenant les étapes consistant à :
a) fournir un matériau de départ ayant la formule structurelle IV, 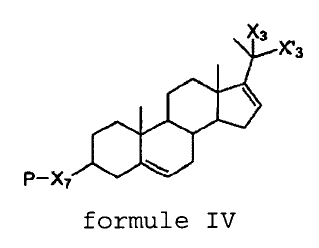 dans lequel X₃, X₃' et X₇ sont sélectionnés parmi le groupe tel qu'indiqué dans l'une quelconque des revendications 1 à 12, et dans lequel P est un groupe de protection,
b) réaliser une réaction entre le composé de l'étape a) et un composé organométallique ayant la formule structurelle V 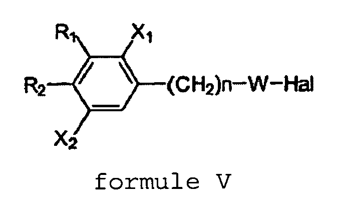 dans lequel X₁, X₂, R₁, R₂ et n sont sélectionnés parmi le groupe tel qu'indiqué dans l'une quelconque des revendications 1 à 12, dans lequel W est un métal ou une combinaison de métaux et dans lequel Hal est un atome d'halogène,
afin que le résultat soit un intermédiaire ayant la formule structurelle III'B 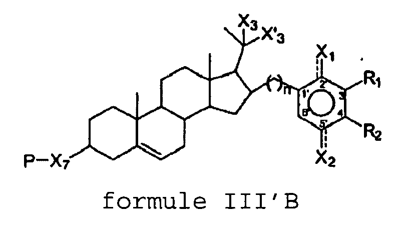 dans lequel X₁, X₂, X₃, X₃', X₇, R₁, R₂ et n sont sélectionnés parmi le groupe tel qu'indiqué dans l'une quelconque des revendications 1 à 12, et dans laquelle p est un groupe de protection,
c) effectuer une réaction entre le composé de l'étape b) avec un composé organométallique ayant la formule structurelle VI
Hal-W-X'₃ formule VI
dans lequel X'₃ est sélectionné parmi le groupe tel qu'indiqué dans l'une quelconque des revendications 1 à 12, dans lequel W est un métal ou une combinaison de métaux, et dans lequel Hal est un atome d'halogène,
afin que le résultat soit un intermédiaire ayant la formule structurelle IIIB 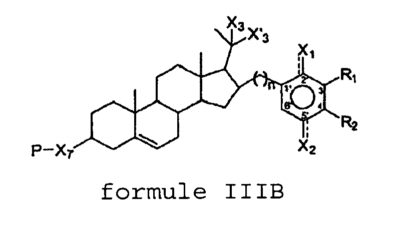 dans lequel X₁, X₂, X₃, X₃', X₇, R₁, R₂ et n sont sélectionnés parmi le groupe tel qu'indiqué dans l'une quelconque des revendications 1 à 12, dans lequel P est un groupe de protection,
d) déprotéger le groupe X₇ du composé obtenu à l'étape c) afin de former un composé ayant la formule structurelle IIB 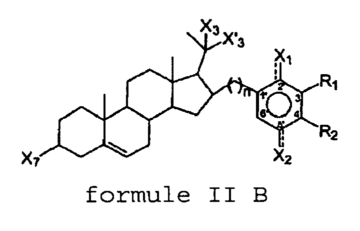 dans lequel X₁, X₂, X₃, X₃', X₇, R₁, R₂ et n sont sélectionnés parmi le groupe tel qu'indiqué dans l'une quelconque des revendications 1 à 12, et
e) oxyder par réaction avec un agent ou des agents oxydants adaptés afin de former un composé de formule IB
ou
e) coupler un glycolsyl dont O est protégé ou un glycosyl non protégé afin de former un composé de formule IIB dans lequel X₁, X₂, X₃, X'₃, X₇, R₁, R₂ et n sont sélectionnés parmi le groupe tel qu'indiqué dans l'une quelconque des revendications 1 à 12 et X₇ est un glycolsyl dont O est protégé ou un glycosyl non protégé, et
f) déprotéger les groupes de glycosyl dont O est protégé afin de former un composé de formule IB dans lequel X₁, X₂, X₃, X'₃, X₄, X₅, X₆, X₇, R₁, R₂ et n sont sélectionnés parmi le groupe tel qu'indiqué dans l'une quelconque des revendications 1 à 12.

14. Composé selon l'une quelconque des revendications 1 à 12 pour une utilisation en tant que médicament.

15. Utilisation d'un composé selon l'une quelconque des revendications 1 à 12 pour la préparation d'un médicament pour le traitement du cancer.

16. Composition pharmaceutique comprenant un excipient acceptable pharmaceutiquement et une quantité efficace thérapeutiquement d'un composé selon l'une quelconque des revendications 1 à 12.
